# EUROPEAN PATENT APPLICATION

(11) **EP 4 541 890 A1**
(43) Date of publication of application: **23.04.2025**
(21) Application number: 23826757.9
(22) Date of filing: 30.03.2023
(51) Int. Cl.: C12N 15/11, C12Q 1/68, C12Q 1/6823, G01N 21/03, G01N 21/64

(54) **METHOD FOR DETECTING FLUORESCENCE**

(30) Priority: 20.06.2022 JP 2022099031; 20.06.2022 JP 2022099032; 20.06.2022 JP 2022099033; 20.06.2022 JP 2022099035
(71) Applicant: Toppan Holdings Inc., Tokyo 110-0016 (JP)
(72) Inventor: OGINO Masayuki, Tokyo 110-0016 (JP); NAGAHARA Misaki, Tokyo 110-0016 (JP); MAKINO Yoichi, Tokyo 110-0016 (JP); HORIUCHI Yosuke, Tokyo 110-0016 (JP); SUZUKI Yuta, Tokyo 110-0016 (JP)
(74) Representative: Plasseraud IP
(86) International application number: PCT/JP2023/013113
(87) International publication number: WO 2023/248568

(57) **Abstract**

A single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the single-stranded nucleotide satisfies one of the following conditions (A) to (C). (A) a single-stranded oligonucleotide including a non-complementary base pair within the hairpin structure. (B) A single-stranded oligonucleotide in which a nucleotide residue labeled with the fluorescent substance and a nucleotide residue labeled with the quenching substance are separated from each other by 4 or more residues. (C) A single-stranded oligonucleotide in which the fluorescent substance is bound to a portion other than the base.

## Description

### [Technical Field]

The present invention relates to methods for detecting fluorescence. The present application is based on and claims the benefit of priority from Japanese Patent Application Nos. 2022-099031, 2022-099032, 2022-099033 and 2022-099035, each filed in Japan on June 20, 2022, the entire contents of all of which are incorporated herein by reference.

### [Background Art]

There are known methods that apply fluorescence resonance energy transfer (FRET) for detecting trace amounts of analyte molecules.

For example, in genetic diagnosis, there are many methods for accurately and quickly detecting and quantifying target nucleic acids. Among these, invasive cleavage assay (ICA) is a method that applies an invader reaction using a nucleic acid cleaving enzyme, such as 5'-nuclease, and FRET, and is excellent in usability and reaction stability (e.g., NPL 1).

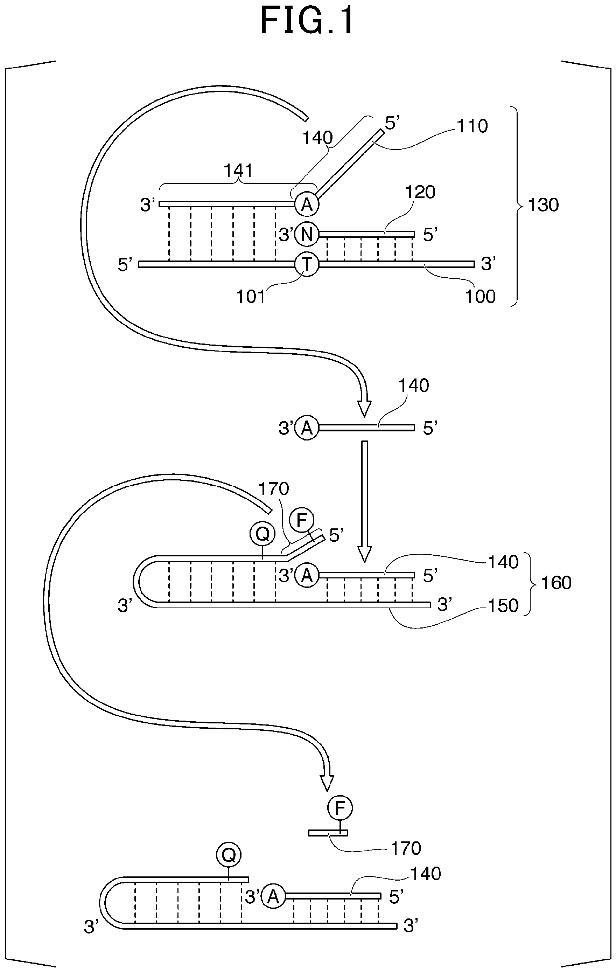

The following description will be given of ICA with reference to Fig. 1. Fig. 1 is a schematic diagram illustrating an example ICA. In the example of Fig. 1, the presence of a T (thymine) base 101 in a target nucleic acid 100 is detected. First, a flap probe 110 and an invasion probe 120 that are complementary to the target nucleic acid 100 are hybridized. As a result, the invasion probe 120 hybridizes to a site of the target nucleic acid 100 adjacent to the position at which the flap probe 110 hybridizes. Then, at least one base at the 3' end of the invasion probe 120 invades the 5' end of a region 141 where the flap probe 110 hybridizes to the target nucleic acid 100, whereby a first triplex structure 130 is formed.

Next, when the first triplex structure 130 is reacted with a flap endonuclease, a flap site 140 of the first triplex structure 130 is cleaved, producing a nucleic acid fragment 140. Then, the nucleic acid fragment 140 hybridizes to a nucleic acid fragment 150, whereby a second triplex structure 160 is formed.

In the example of Fig. 1, a fluorescent substance F is bound to the 5' end of the nucleic acid fragment 150, and a quenching substance Q is bound to the nucleic acid fragment 150 a few bases from the 5' end to the 3' side. The fluorescent substance F is located in spatial proximity to the quenching substance Q. Therefore, fluorescence emitted by the fluorescent substance F is quenched by the quenching substance Q.

Then, when the second triplex structure 160 is reacted with a flap endonuclease, a flap site 170 of the second triplex structure 160 is cleaved, producing a nucleic acid fragment 170. As a result, the fluorescent substance F is released from the quenching substance Q, and emits fluorescence when irradiated with excitation light. By detecting this fluorescence, the presence of the T (thymine) base 101 in the target nucleic acid 100 can be detected.

In this specification, the flap probe may also be called an allele probe. Further, the invasion probe may also be called an invader oligo.

The ICA described in NPL 1 uses a FRET cassette including a fluorescent substance and a quenching substance. When no target nucleic acid is present in the reaction system, a fluorescent substance is in spatial proximity to the quenching substance. Accordingly, fluorescence emission from the fluorescent substance is suppressed by the quenching substance. When a target nucleic acid is present in the reaction system, ICA reaction proceeds due to the nucleic acid cleaving enzyme to thereby separate the fluorescent substance from the quenching substance. As a result, fluorescence is emitted from the fluorescent substance.

In ICA, it is possible to simultaneously detect a plurality of types of target nucleic acids by using a plurality of types of probes with different fluorescence wavelengths corresponding to the plurality of types of target nucleic acids. For example, when fluorescence has five colors, i.e., blue, green, yellow, orange and red, five different types of target nucleic acids can be simultaneously detected in a single measurement.

Further, when ICA is performed in a microspace such as microwells, the concentration of the analyte molecules in the reaction system can be apparently concentrated. This can shorten the time required for the detection signal to reach a sufficient intensity in the microspace. For example, PTL 1 describes that genetic testing is possible by performing enzyme reactions in a microspace with a volume of 1 pL or less.

### [Citation List]

### [Patent Literature]

PTL 1: JP 2004-309405 A

### [Non-Patent Literature]

NPL 1: Eis et al, An invasive cleavage assay for direct quantitation of specific RNAs, Nature Biotechnology, Vol. 19, 673-676 (2001).

### [Summary of Invention]

### [Technical Problem]

The present invention has been made to provide a technique for improving the reaction efficiency in reactions for detecting trace amounts of analyte molecules.

For example, when a plurality of types of fluorescent probes are used, detecting fluorescence is difficult since the intensity of blue and red fluorescence is weak due to the characteristics of fluorescent molecules. Therefore, in one aspect, the present invention provides a detection method capable of further improving the fluorescence intensity emitted from a fluorescent probe including a fluorescent substance and a quenching substance in the presence of analyte molecules.

Further, the inventors of the present invention have found that, in ICA, formation of a triplex structure by hybridization of the nucleic acid fragment 140 and the nucleic acid fragment 150 (hereinafter, also referred to as a "fluorescent substrate") shown in Fig. 1 may be inhibited by the fluorescent substance F and the quenching substance Q labeled to the fluorescent substrate. If the formation of a triplex structure is inhibited, the reactivity in ICA is reduced. Therefore, in one aspect, the present invention provides a fluorescent substrate for ICA capable of efficiently forming a triplex structure.

Further, the inventors of the present invention have found that, in ICA illustrated in Fig. 1, background signals may increase in addition to genetic polymorphism- or gene mutationspecific signals. An increase in background signals leads to false-positive judgement, which is a risk factor for erroneous judgement in genetic diagnosis. Therefore, in one aspect, the present invention provides a technique for suppressing an increase in background in ICA.

Further, the inventors of the present invention have found that, in ICA illustrated in Fig. 1, there is room for improvement in the efficiency of cleavage of the fluorescent substrate by the flap endonuclease. Therefore, in one aspect, the present invention provides a technique for improving the efficiency of cleavage of the fluorescent substrate by the flap endonuclease in ICA.

### [Solution to Problems]

The present invention includes the following aspects.
[1] A single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the single-stranded nucleotide satisfies one of the following conditions (A) to (C):
   (A) a single-stranded oligonucleotide including a non-complementary base pair within the hairpin structure;
   (B) a single-stranded oligonucleotide in which a nucleotide residue labeled with the fluorescent substance and a nucleotide residue labeled with the quenching substance are separated from each other by 4 or more residues; and
   (C) a single-stranded oligonucleotide in which the fluorescent substance is bound to a portion other than the base.
[2] The single-stranded oligonucleotide according to [1], which satisfies the condition (A), wherein the fluorescent substance is labeled at a 5' end nucleotide residue, and the quenching substance is labeled at a nucleotide residue located closer to the 5' side among nucleotide residues constituting the non-complementary base pair.
[3] The single-stranded oligonucleotide according to [1], wherein the base of the 5' end nucleotide residue is a pyrimidine base.
[4] The single-stranded oligonucleotide according to [1], wherein the quenching substance is labeled on the 3' side of the fluorescent substance, and an oligonucleotide fragment containing the fluorescent substance is released when cleaved by a flap endonuclease.
[5] The single-stranded oligonucleotide according to [1], wherein the fluorescent substance is labeled at a 5' end nucleotide residue, an oligonucleotide fragment containing the fluorescent substance is released when cleaved by a flap endonuclease, and the oligonucleotide fragment has a length of 2 or more residues.
[6] The single-stranded oligonucleotide according to [1], wherein a phosphate group is added to the 5' end, and the fluorescent substance is bound to the phosphate group.
[7] The single-stranded oligonucleotide according to [1], wherein the quenching substance is bound to a base of a nucleotide residue located on the 3' side of the fluorescent substance.
[8] The single-stranded oligonucleotide according to [1], wherein the fluorescent substance has a molecular weight of 350 to 1,100.
[9] The single-stranded oligonucleotide according to [1], wherein both the fluorescent substance and the quenching substance are present in one strand of a double-stranded oligonucleotide portion of the hairpin structure.
[10] A method for detecting fluorescence from a substrate including a fluorescent substance, a quenching substance and a substrate body, the method including: a step of causing the fluorescent substance to emit fluorescence by separating the fluorescent substance from the quenching substance in the substrate, wherein when the fluorescent substance is in spatial proximity to the quenching substance, fluorescence emission from the fluorescent substance is suppressed by the quenching substance, and a peak wavelength of an absorption spectrum of the quenching substance is on a shorter wavelength side of a peak wavelength of an emission spectrum of the fluorescent substance.
[11] The method according to [10], wherein the step of causing the fluorescent substance to emit fluorescence is performed in the presence of a target substance.
[12] The method according to [10] or [11], wherein the peak wavelength of the absorption spectrum of the quenching substance is 50 nm to 100 nm shorter than the peak wavelength of the emission spectrum of the fluorescent substance.
[13] The method according to [10] or [11], wherein the substrate body is a nucleic acid.
[14] The method according to [10] or [11], wherein the peak wavelength of the emission spectrum of the fluorescent substance is 600 nm to 700 nm, and the peak wavelength of the absorption spectrum of the quenching substance is 550 nm to 640 nm.
[15] The method according to [14], wherein in the step of causing the fluorescent substance to emit fluorescence, the fluorescent substance is separated from the quenching substance in the substrate by the action of a nucleic acid cleaving enzyme.
[16] The method according to [10] or [11], wherein the step of causing the fluorescent substance to emit fluorescence is performed in a microspace.
[17] The method according to [11], wherein the target substance is a nucleic acid.
[18] The method according to [10] or [11], further including a step of observing the fluorescence with a fluorescence microscope.

The present invention may be construed to include the following aspects.
[A1] A method for detecting fluorescence from a substrate including a fluorescent substance, a quenching substance and a substrate body, the method including: a step of causing the fluorescent substance to emit fluorescence by separating the fluorescent substance from the quenching substance in the substrate, wherein when the fluorescent substance is in spatial proximity to the quenching substance, fluorescence emission from the fluorescent substance is suppressed by the quenching substance, and a peak wavelength of an absorption spectrum of the quenching substance is on a shorter wavelength side of a peak wavelength of an emission spectrum of the fluorescent substance.
[A2] The method according to [A1], wherein the step of causing the fluorescent substance to emit fluorescence is performed in the presence of a target substance.
[A3] The method according to [A1] or [A2], wherein the peak wavelength of the absorption spectrum of the quenching substance is 50 nm to 100 nm shorter than the peak wavelength of the emission spectrum of the fluorescent substance.
[A4] The method according to any one of [A1] to [A3], wherein the substrate body is a nucleic acid.
[A5] The method according to any one of [A1] to [A4], wherein the peak wavelength of the emission spectrum of the fluorescent substance is 600 nm to 700 nm, and the peak wavelength of the absorption spectrum of the quenching substance is 550 nm to 640 nm.
[A6] The method according to [A4], wherein in the step of causing the fluorescent substance to emit fluorescence, the fluorescent substance is separated from the quenching substance in the substrate by the action of a nucleic acid cleaving enzyme.
[A7] The method according to any one of [A1] to [A6], wherein the step of causing the fluorescent substance to emit fluorescence is performed in a microspace.
[A8] The method according to [A2], wherein the target substance is a nucleic acid.
[A9] The method according to any one of [A1] to [A8], further including a step of observing the fluorescence with a fluorescence microscope.

The present invention may be construed to include the following aspects.
[B1] A single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the fluorescent substance is bound to a portion other than the base.
[B2] The single-stranded oligonucleotide according to [B1], wherein a phosphate group is added to the 5' end, and the fluorescent substance is bound to the phosphate group.
[B3] The single-stranded oligonucleotide according to [B2], wherein the quenching substance is bound to a base of a nucleotide residue located on the 3' side of the fluorescent substance.
[B4] The single-stranded oligonucleotide according to any one of [B1] to [B3], wherein the base of the 5' end nucleotide residue is a pyrimidine base.
[B5] The single-stranded oligonucleotide according to any one of [B1] to [B4], wherein the quenching substance is labeled on the 3' side of the fluorescent substance, and an oligonucleotide fragment containing the fluorescent substance is released when cleaved by a flap endonuclease.
[B6] The single-stranded oligonucleotide according to any one of [B1] to [B5], wherein the fluorescent substance has a molecular weight of 350 to 1,100.
[B7] The single-stranded oligonucleotide according to any one of [B1] to [B6], wherein both the fluorescent substance and the quenching substance are present in one strand of a double-stranded oligonucleotide portion of the hairpin structure.
[B8] A method for improving the efficiency of forming a triplex structure in a fluorescent substrate for flap endonuclease, the fluorescent substrate being a first single-stranded oligonucleotide labeled with a fluorescent substance and a quenching substance, the method including a step of bringing the first single-stranded oligonucleotide into contact with a second single-stranded oligonucleotide having a complementary base sequence on the 3' side of the first single-stranded oligonucleotide, wherein the first single-stranded oligonucleotide forms a hairpin structure by self-hybridization on the 5' side, and when the second single-stranded oligonucleotide hybridizes, a triplex structure is formed on the 5' end portion, and cleaved by the flap endonuclease, whereby the fluorescent substance is released from the quenching substance, the fluorescent substance emitting fluorescence when irradiated with excitation light, and the fluorescent substance is bound to a portion other than the base of the first single-stranded oligonucleotide.

The present invention may be construed to include the following aspects.
[C1] A single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the single-stranded oligonucleotide includes a non-complementary base pair within the hairpin structure.
[C2] The single-stranded oligonucleotide according to [C1], wherein the fluorescent substance is labeled at a 5' end nucleotide residue, and the quenching substance is labeled at a nucleotide residue located closer to the 5' side among nucleotide residues constituting the non-complementary base pair.
[C3] The single-stranded oligonucleotide according to [C1] or [C2], wherein the base of the 5' end nucleotide residue is a pyrimidine base.
[C4] The single-stranded oligonucleotide according to any one of [C1] to [C3], wherein the quenching substance is labeled on the 3' side of the fluorescent substance, and an oligonucleotide fragment containing the fluorescent substance is released when cleaved by a flap endonuclease.
[C5] A method for suppressing an increase in background in ICA, the method including a step of adding a fluorescent substrate to a reaction solution for ICA, wherein the fluorescent substrate is a single-stranded oligonucleotide labeled with a fluorescent substance and a quenching substance, the single-stranded oligonucleotide forms a hairpin structure by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the single-stranded oligonucleotide includes a non-complementary base pair within the hairpin structure.

The present invention may be construed to include the following aspects.
[D1] A single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and a nucleotide residue labeled with the fluorescent substance and a nucleotide residue labeled with the quenching substance are separated from each other by 4 or more residues.
[D2] The single-stranded oligonucleotide according to [D1], wherein the fluorescent substance is labeled at a 5' end nucleotide residue, and an oligonucleotide fragment containing the fluorescent substance is released when cleaved by a flap endonuclease.
[D3] The single-stranded oligonucleotide according to [D2], wherein the oligonucleotide fragment has a length of 2 or more residues.
[D4] The single-stranded oligonucleotide according to [D2] or [D3], wherein the base of the 5' end nucleotide residue is a pyrimidine base.
[D5] The single-stranded oligonucleotide according to any one of [D2] to [D4], wherein the single-stranded oligonucleotide includes a non-complementary base pair within the hairpin structure, and the quenching substance is labeled at a nucleotide residue located closer to the 5' side among nucleotide residues constituting the non-complementary base pair.
[D6] A method for improving cleavage efficiency in cleavage of a nucleic acid triplex structure with a flap endonuclease, the method including a step of bringing the flap endonuclease into contact with the nucleic acid triplex structure, wherein the nucleic acid triplex structure includes a single-stranded oligonucleotide labeled with a fluorescent substance and a quenching substance, and a single-stranded oligonucleotide having a complementary base sequence on the 3' side, the single-stranded oligonucleotide forms a hairpin structure by self-hybridization on the 5' side, and when the single-stranded oligonucleotide having a complementary base sequence on the 3' side hybridizes, a triplex structure is formed on the 5' end portion, and cleaved by the flap endonuclease, whereby the fluorescent substance is released from the quenching substance, the fluorescent substance emitting fluorescence when irradiated with excitation light, and a nucleotide residue labeled with the fluorescent substance and a nucleotide residue labeled with the quenching substance are separated from each other by 4 or more residues.

### [Advantageous Effects of Invention]

According to the present invention, it is possible to provide a technique for improving the reaction efficiency in reactions for detecting trace amounts of analyte molecules.

In one aspect, the present invention can provide a detection method capable of further improving the fluorescence intensity emitted from a fluorescent probe including a fluorescent substance and a quenching substance in the presence of analyte molecules. This detection method is suitable for use in digital measurements using ICA.

In one aspect, the present invention can provide a fluorescent substrate for ICA capable of efficiently forming a triplex structure.

In one aspect, the present invention can provide a technique for suppressing an increase in background in ICA.

In one aspect, the present invention can provide a technique for improving the efficiency of cleavage of the fluorescent substrate by the flap endonuclease in ICA.

### [Brief Description of Drawings]

Fig. 1 is a schematic diagram illustrating an example of an invasive cleavage assay (ICA).
Fig. 2 is a schematic cross-sectional view illustrating a method for supplying a reagent solution to a fluidic device.
Fig. 3 is a diagram schematically illustrating a first cleavage structure.
Fig. 4 is a diagram schematically illustrating a second α-cleavage structure.
Fig. 5 is a diagram schematically illustrating a second β-cleavage structure.
Fig. 6 is a diagram schematically illustrating a reaction mechanism of ICA in Experimental Example A1.
Fig. 7 is a photograph of fluorescence when FRET cassette 4-1 is used in Experimental Example A1.
Fig. 8 is a photograph of fluorescence when FRET cassette 4-2 is used in Experimental Example A1.
Fig. 9 is a photograph of fluorescence when FRET cassette 4-3 is used in Experimental Example A1.
Fig. 10 is a histogram of signal intensity when FRET cassette 4-1 is used in Experimental Example A1.
Fig. 11 is a histogram of signal intensity when FRET cassette 4-2 is used in Experimental Example A1.
Fig. 12 is a histogram of signal intensity when FRET cassette 4-3 is used in Experimental Example A1.
Fig. 13 is a graph showing an S/N value in Experimental Example A1.
Fig. 14 is a graph showing an S-N value in Experimental Example A1.
Fig. 15 is a graph showing the results when a fluorescent substrate in which a fluorescent substance is bound to a base was used in Experimental Example A1.
Fig. 16 is a graph showing the results when a fluorescent substrate in which a fluorescent substance is bound to a moiety other than the base was used in Experimental Example A1.
Fig. 17 is a graph showing the results of Experimental Example B1.
Fig. 18 is a graph showing the results of Experimental Example B1.
Fig. 19 is a graph showing the results of Experimental Example B2.
Fig. 20 is a graph showing the results of Experimental Example B2.
Fig. 21 is a graph showing the results of Experimental Example B3.
Fig. 22 is a graph showing the results of Experimental Example B3.
Fig. 23 is a graph showing the results of Experimental Example B4.
Fig. 24 is a graph showing the results of Experimental Example B4.
Fig. 25 is a graph showing the results of Experimental Example B5.
Fig. 26 is a graph showing the results of Experimental Example C1.
Fig. 27 is a graph showing the results of Experimental Example C1.
Fig. 28 is a graph showing the results of Experimental Example D1.
Fig. 29 is a graph showing the results of Experimental Example D1.
Fig. 30 is a graph showing the results of Experimental Example D1.
Fig. 31 is a graph showing the results of Experimental Example D1.

### [Description of Embodiments]

### <Embodiment A>

### [Method for Detecting Fluorescence from Substrate Including Fluorescent Substance, Quenching Substance and Substrate Body]

A method according to a first aspect is a method for detecting fluorescence from a substrate including a fluorescent substance, a quenching substance and a substrate body. The method according to the first aspect includes a step of causing the fluorescent substance to emit fluorescence by separating the fluorescent substance from the quenching substance in the substrate.

### (Substrate)

The substrate includes a substrate body, a fluorescent substance and a quenching substance. The fluorescent substance and the quenching substance are each bound to the substrate body.

The bonding between the fluorescent substance and the substrate body is not particularly limited as long as the advantageous effects of the present invention are achieved, and may be chemical bonding. The bonding between the quenching substance and the substrate body is not particularly limited as long as the advantageous effects of the present invention are achieved, and may be chemical bonding.

The substrate body is not particularly limited as long as the advantageous effects of the present invention are achieved, and may be natural or synthetic. Examples of the substrate body include polymers. The polymers are not particularly limited, and examples thereof include nucleic acids, nucleic acid analogues, polyamino acids, proteins and oligoamino acids.

Examples of the natural nucleic acids include genomic DNA, mRNA, rRNA, hnRNA, miRNA and tRNA. The natural nucleic acids may be those recovered from living organisms or may be those recovered from water, organic matter, or the like that has been in contact with living organisms. Methods for recovering natural nucleic acids include known methods such as the phenol/chloroform method.

Examples of the synthetic nucleic acids include synthetic DNA, synthetic RNA, cDNA, bridged nucleic acid (BNA) and locked nucleic acid (LNA).

Methods for synthesizing the nucleic acid are not particularly limited, and examples thereof include known chemical synthesis methods such as the β-cyanoethyl phosphoramidite and DNA solid-phase synthesis, known nucleic acid amplification methods and reverse transcription reactions. Examples of the nucleic acid amplification methods include PCR, LAMP, SMAP, NASBA and RCA.

### (Fluorescent Substance)

Examples of the fluorescent substance include red fluorescent substances such as ATTO 643, ATTO 633, Alexa Fluour 647, Cy5, HiLyte Fluor 647 and ATTO 663.

The peak wavelength of the fluorescence spectrum of the fluorescent substance is preferably 600 nm to 700 nm, more preferably 610 nm to 690 nm, still more preferably 620 nm to 680 nm, particularly preferably 630 nm to 670 nm, and most preferably 640 nm to 660 nm.

### (Quenching Substance)

Examples of the quenching substance include Black Hole Quencher (BHQ) (registered trademark)-1, BHQ (registered trademark)-2, BHQ (registered trademark)-3, Tide Quencher 1 (TQ1), Tide Quencher 2 (TQ2), Tide Quencher 2WS (TQ2WS), Tide Quencher 3 (TQ3), Tide Quencher 3WS (TQ3WS), Tide Quencher 4 (TQ4), Tide Quencher 4WS (TQ4WS), Tide Quencher 5 (TQ5), Tide Quencher 5WS (TQ5WS), Tide Quencher 6WS (TQ6WS), Tide Quencher 7WS (TQ7WS), QSY35, QSY7, QSY9, QSY21, Iowa Black FQ and Iowa Black RQ, and it is preferred to use one or more selected from the group consisting of BHQ-2, TQ4, TQ4WS, TQ5, TQ5WS, QSY21 and Iowa Black RQ, and more preferred to use BHQ-2.

In one aspect, the peak wavelength of the absorption spectrum of the quenching substance is preferably 550 nm to 640 nm, more preferably 560 nm to 630 nm, still more preferably 570 nm to 620 nm, and particularly preferably 570 nm to 600 nm.

In one aspect, the peak wavelength of the absorption spectrum of the quenching substance is on the shorter wavelength side of the peak wavelength of the emission spectrum of the fluorescent substance. In this case, the peak wavelength of the absorption spectrum of the quenching substance is preferably 50 nm to 100 nm shorter than the peak wavelength of the emission spectrum of the fluorescent substance, more preferably 55 nm to 80 nm shorter, and still more preferably 60 nm to 70 nm shorter.

When the fluorescent substance is in spatial proximity to the quenching substance in the substrate, fluorescence emission from the fluorescent substance is suppressed by the quenching substance. Here, the "fluorescence emission is suppressed" means as follows.

In the absence of the quenching substance, the intensity of fluorescence emitted from the fluorescent substance when the fluorescent substance is irradiated with excitation light is defined as A. Further, in the presence of the quenching substance in spatial proximity to the fluorescent substance, the intensity of fluorescence emitted from the fluorescent substance when the fluorescent substance is irradiated with excitation light is defined as B. Here, the "fluorescence emission is suppressed" means that the value of B/A is 40% or less.

When the fluorescent substance is in spatial proximity to the quenching substance, the distance between the fluorescent substance and the quenching substance is not particularly limited as long as the fluorescence emitted from the fluorescent substance is suppressed by the quenching substance, but is preferably 10 nm or less, more preferably 5 nm or less, and still more preferably 2 nm or less.

A method of separating fluorescent substance from the quenching substance in the substrate is not particularly limited as long as fluorescence can be emitted from the fluorescent substance, and the method may be, for example, subjecting the substrate having the fluorescent substance and the quenching substance to a physical action such as shearing, heating, cooling or electromagnetic irradiation, a chemical action such as exposure to a chemical substance, or a biological action such as an enzymatic reaction.

When the fluorescent substance is separated from the quenching substance, the distance between the fluorescent substance and the quenching substance is not particularly limited as long as fluorescence can be emitted from the fluorescent substance, but is preferably 5 nm or greater, more preferably 10 nm or greater, and still more preferably 20 nm or greater.

### (Target Substance)

A step of causing the fluorescent substance to emit fluorescence may be performed in the presence of a target substance. The target substance is not particularly limited as long as it directly or indirectly separates the fluorescent substance from the quenching substance, but examples thereof include nucleic acids, enzymes and proteins such as antibodies, and nucleic acids are preferred.

When the target substance is a nucleic acid, the fluorescent substance can be separated from the quenching substance in the substrate by the action of a nucleic acid cleaving enzyme in the step of causing the fluorescent substance to emit fluorescence.

### (Microspace)

In the method according to the first aspect, the step of causing the fluorescent substance to emit fluorescence is preferably performed in a microspace. Specifically, it is preferred to use a device including a well array having a plurality of microwells. According to conventional methods, since the absolute number of substrates is small in a microspace, the number of fluorescent molecules as the products of cleavage reaction is also small, reducing the fluorescence intensity. On the other hand, the method of the present embodiment can improve the reaction efficiency and thus increase the number of fluorescent molecules as the products of cleavage. Therefore, the fluorescence intensity can be enhanced even in a microspace. Examples of the device that can be used include, but are not limited to, those described below.

Although the shape, size and arrangement of wells are not specifically limited, it is preferred to use a well array composed of wells that can accommodate liquid containing the target nucleic acid used in a method according to the present invention and a predetermined amount of reagent solution or the like for use in the detection step. The wells may be used without any treatment, or may be pre-treated such as by immobilizing an extraction reagent, a detection reagent such as antibody, a specific binding substance, or the like on the inner wall of the wells, or covering the well openings with a lipid bilayer depending on the purpose.

The device may have flow channels, and liquid in which the target nucleic acid is dispersed may be supplied through the flow channels. Although the shape, structure, volume, or the like of the flow channels is not specifically limited, it is preferred to use a device having flow channels configured such that, when a dispersion liquid of a structure is supplied, the structure is introduced into respective wells of the well array, and, when a sealing solution is inserted, the respective wells are individually sealed to thereby form microdroplets.

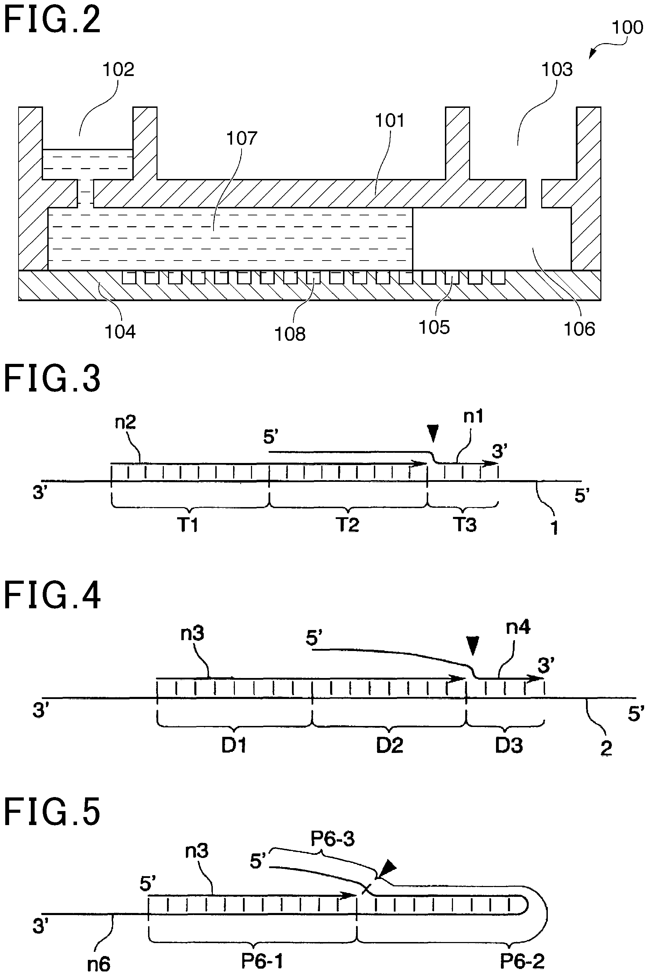

Fig. 2 is a schematic cross-sectional view of a device according to one aspect. As shown in Fig. 2, a device 100 includes a substrate member 104 and a lid member 101.

The substrate member 104 may be made of a light-transmitting resin. The substrate member 104 may be substantially transparent.

The substrate member 104 includes a plurality of wells 105. The wells 105 of the substrate member 104 are open to the surface of the substrate member 104. The shape, dimensions and arrangement of the wells 105 are not particularly limited. According to the example shown in Fig. 2, in the device 100, a plurality of wells 105 having the same shape and size capable of accommodating a reagent solution 107 (liquid in which the target nucleic acid is dispersed) are formed in the substrate member 104. Further, when particles are used in the method according to the first aspect, the wells 105 having the same shape and size capable of accommodating one or more particles and accommodating a predetermined amount of the reagent solution 107 containing the particles may be formed in the substrate member 104.

In the device 100, the wells 105 may have, for example, a diameter of approximately 3 µm and a depth of approximately 4.5 µm. Further, the wells 105 may be arranged in a triangular lattice or a square lattice in the substrate member 104.

A region of the substrate member 104 including the plurality of wells 105 is a region to be filled with the reagent solution 107, which is a target of analysis. Inside this region, a flow channel 106 is provided between the substrate member 104 and the lid member 101.

The lid member 101 may be welded or bonded to the substrate member 104. For example, the lid member 101 may be made of a thermoplastic resin such as a cycloolefin polymer or a cycloolefin copolymer.

The substrate member 104 may be formed of, for example, a resin. Although the type of the resin is not specifically limited, it is preferred to use a resin that is resistant to a reagent and a sealing solution used in forming droplets. Further, in fluorescence observation of a signal, it is preferred to select a resin with low autofluorescence. Examples of the material include cycloolefin polymer, cycloolefin copolymer, silicone, polypropylene, polycarbonate, polystyrene, polyethylene, polyvinyl acetate, fluororesin and amorphous fluororesin. The materials described as examples of the substrate member 104 are merely illustrative, and the material is not limited thereto.

The substrate member 104 may have a plurality of wells 105 on one surface in the thickness direction. A forming method using a resin may be thermal imprinting or optical imprinting as well as injection molding. In addition, when a fluororesin is used, for example, a layer of CYTOP (registered trademark) (AGC Inc.) is formed on the substrate member 104, and fine apertures formed in the CYTOP (registered trademark) may serve as the wells 105.

The lid member 101 is formed to have a projection on a surface that faces the substrate member 104 when assembled. For example, a fluid of a thermoplastic resin may be formed with a mold into a plate shape having a projection. In addition, the lid member 101 may be formed to have a liquid supply port 102 and a liquid discharge port 103.

Once the lid member 101 and the substrate member 104 are formed as described above, the lid member 101 and the substrate member 104 are stacked with the projection of the lid member being in contact with the surface of the substrate member 104 to which the wells 105 are open. Further, the lid member 101 and the substrate member 104 are welded to each other by laser welding or the like while being stacked as described above.

The number of wells included in the device is preferably 100,000 to 6,000,000. The total volume of the wells is preferably 0.1 µL to 10 µL.

### (ICA)

The method according to the first aspect can be applied to, for example, an invasive cleavage assay (ICA).

The target substance in the method according to the first aspect may be a target nucleic acid in ICA. In ICA, two types of oligonucleotides capable of hybridizing to a target nucleic acid are used to form a cleavage structure having a specific overlapping structure. Then, the obtained cleavage structure is cleaved with an enzyme having 5' nuclease activity, and the target nucleic acid is detected by detecting the cleavage fragment.

More specifically, ICA is a method described below. First, two types of oligonucleotides are each annealed to a target nucleic acid at different sites adjacent to each other so that 5' side of one of the oligonucleotides overlaps the other of the oligonucleotides annealed to the target nucleic acid to thereby form a cleavage structure having a specific overlapping structure.

**In** the cleavage structure, a part of one of the oligonucleotides invades under the other of the oligonucleotides. The invading oligonucleotide is also called an invader oligo, and the overlapping oligonucleotide is also called an allele probe.

The invasion structure in the cleavage structure is recognized by an enzyme having 5' nuclease activity, and the overlapping site on the 5' side of the allele probe is cleaved. By designing the allele probe to be cleaved to have a melting point close to the cleavage reaction temperature of the 5' nuclease activity, the site annealed to the target nucleic acid that remains after cleavage also dissociates from the target nucleic acid. Then, when an uncleaved allele probe is newly annealed, a cleavage structure is formed again, whereby the cleavage reaction is repeated, amplifying the cleaved fragments (flap fragments). Therefore, by measuring the flap fragments, the target nucleic acid can be detected.

Various methods for measuring flap fragments are disclosed. For example, as a secondary reaction, a flap fragment as an invader oligo and other oligonucleotide as an allele probe can be annealed to a detection nucleic acid different from the target nucleic acid to form a cleavage structure, and the allele probe can be cleaved in the same manner as in the primary reaction to amplify the secondary flap fragments, which can then be detected.

**In** the secondary reaction, measurement can be performed using a fluorescence resonance energy transfer (FRET) reaction. Specifically, the flap fragment acts as an invader oligo on a FRET cassette (probe having a fluorescent substance and a quenching substance) to form a cleavage structure, the FRET cassette is cleaved to separate the fluorescent substance from the quenching substance, and the resulting fluorescence signal can be measured measure the flap fragments with high sensitivity. The measurement reaction of the flap fragments can proceed simultaneously in the same reaction solution as the production reaction of the flap fragments.

When ICA is performed in the wells of the above device, it is preferred that each well of the device contains one or less target substance.

The method according to the present aspect may further include a step of observing the fluorescence with a fluorescence microscope.

According to the method of the first aspect described above, the fluorescence intensity emitted from a fluorescent probe including a fluorescent substance and a quenching substance is further enhanced in the presence of analyte molecules.

An embodiment of the method according to the first aspect may be, for example, the following embodiment. A method according to the present embodiment is a method in which the substrate described above in the first aspect is applied to the ICA reaction illustrated in Fig. 1.

In the method according to the present embodiment, "the step of causing the fluorescent substance to emit fluorescence by separating the fluorescent substance from the quenching substance in the substrate" in the method according to the first aspect includes a step of bringing a first single-stranded oligonucleotide labeled with a fluorescent substance and a quenching substance into contact with a second single-stranded oligonucleotide having a complementary base sequence on the 3' side of the first single-stranded oligonucleotide.

In the method according to the present embodiment, the first single-stranded oligonucleotide forms a hairpin structure by self-hybridization on the 5' side, and when the second single-stranded oligonucleotide hybridizes, a triplex structure is formed on the 5' end portion, and cleaved by a nucleic acid cleaving enzyme, whereby the fluorescent substance is separated from the quenching substance, the fluorescent substance emitting fluorescence when irradiated with excitation light.

In the method according to the present embodiment, when the fluorescent substance is in spatial proximity to the quenching substance, fluorescence emission from the fluorescent substance is suppressed by the quenching substance.

In the method according to the present embodiment, the peak wavelength of the absorption spectrum of the quenching substance is on the shorter wavelength side of the peak wavelength of the emission spectrum of the fluorescent substance.

The method according to the present embodiment may include a step of detecting fluorescence. The fluorescence may be detected with a fluorescence microscope.

The first single-stranded oligonucleotide having a hairpin structure by self-hybridization on the 5' side and labeled with a fluorescent substance and a quenching substance corresponds to the nucleic acid fragment 150 in Fig. 1. The second single-stranded oligonucleotide having a complementary base sequence on the 3' side of the first single-stranded oligonucleotide corresponds to the nucleic acid fragment 140 derived from the flap site 140 in Fig. 1. The triplex structure formed on the 5' end portion corresponds to the second triplex structure 160 in Fig. 1.

As described later in the examples, due to the fluorescent substance of the fluorescent substrate being bound to a portion other than a base, a triplex structure can be efficiently formed, improving the reactivity in ICA. The term "fluorescent substance being bound to a portion other than a base" means that the fluorescent substance is bound to a sugar residue or a phosphate group, among sugar residues, phosphate groups and bases of the oligonucleotide constituting the fluorescent substrate. In particular, it is preferred that the fluorescent substance is bound to a phosphate group of the fluorescent substrate, since this further enhances the fluorescence intensity.

In this specification, the term "reactivity in ICA is improved" may mean that the fluorescence signal detected from the result of ICA reaches a predetermined value in a shorter period of time. Alternatively, it may mean that the fluorescence signal intensity detected from the result of ICA reaches a higher value. Alternatively, it may mean that the background fluorescence signal detected from the result of ICA is maintained at a lower value.

The single-stranded oligonucleotide of the present embodiment preferably has a length of approximately 20 bases to 150 bases. Further, the number of base pairs constituting the hairpin structure is preferably approximately 5 to 50.

In the single-stranded oligonucleotide labeled with a fluorescent substance and a quenching substance, the number of bases between the nucleic acid to which the fluorescent substance is bound and the nucleic acid to which the quenching substance is bound is preferably 11 bases or greater and 30 base or less, more preferably 4 bases or greater and 10 bases or less, and still more preferably 1 base or greater and 3 bases or less.

The nucleic acid cleaving enzyme is preferably an enzyme having 5' nuclease activity, more preferably a modified DNA polymerase having 5' nuclease activity but lacking polymerase activity, and still more preferably a flap endonuclease.

Examples of the flap endonuclease include flap endonuclease 1 (NCBI accession number: WP_011012561.1, Holliday junction 5' flap endonuclease (GEN1) (NCBI accession number: NP_001123481.3), excision repair protein (NCBI accession number: AAC37533.1), and the like.

In the single-stranded oligonucleotide labeled with a fluorescent substance and a quenching substance (also referred to as a fluorescent substrate), a phosphate group is preferably added to the 5' end, and the fluorescent substance is preferably bound to the phosphate group added to the 5' end.

As described later in the examples, a fluorescent substrate in which a phosphate group is added to the 5' end and a fluorescent substance is bound to the phosphate group added to the 5' end has improved reactivity in ICA and thus efficiently forms a triplex structure compared to a fluorescent substrate in which a fluorescent substance is bound to a base.

The following formula (A1) is a chemical formula showing an example fluorescent substrate in which a phosphate group is added to the 5' end and a fluorescent substance is bound to the phosphate group added to the 5' end. As shown in the following formula (A1), the fluorescent substance may be bound to the phosphate group added to the 5' end via a linker containing a hydrocarbon group having approximately 1 to 100 carbon atoms. The linker preferably has approximately 1 to 30 carbon atoms, and more preferably approximately 1 to 10 carbon atoms.

A linker having the number of carbon atoms equal to or greater than the lower limit can maintain an appropriate distance between the fluorescent substance and the site cleaved by the nucleic acid cleaving enzyme. As a result, a cleavage reaction by the nucleic acid cleaving enzyme is less likely to be inhibited by the fluorescent substance, improving the reaction efficiency by the nucleic acid cleaving enzyme. A linker having the number of carbon atoms equal to or smaller than the upper limit can reduce the distance between the fluorescent substance and the quenching substance. As a result, the quenching effect of the quenching substance is improved.

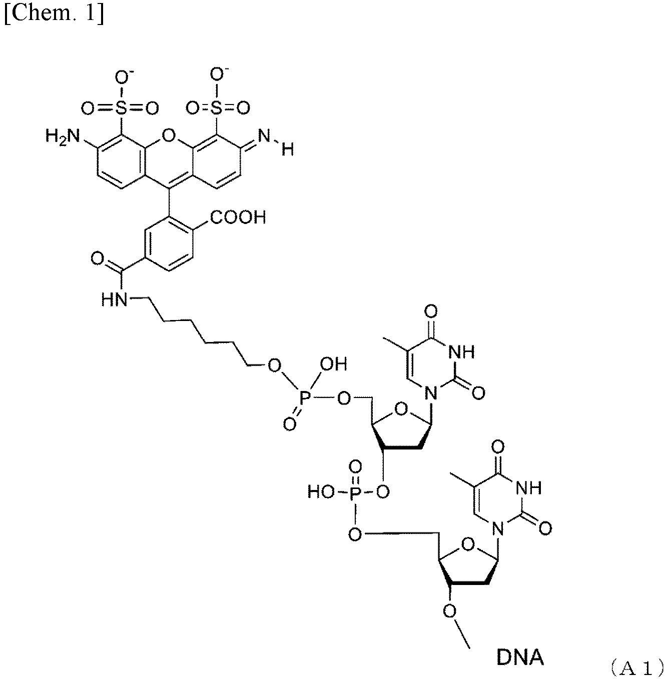

The following formula (A2) is a chemical formula showing an example fluorescent substrate in which a fluorescent substance is bound to a base.

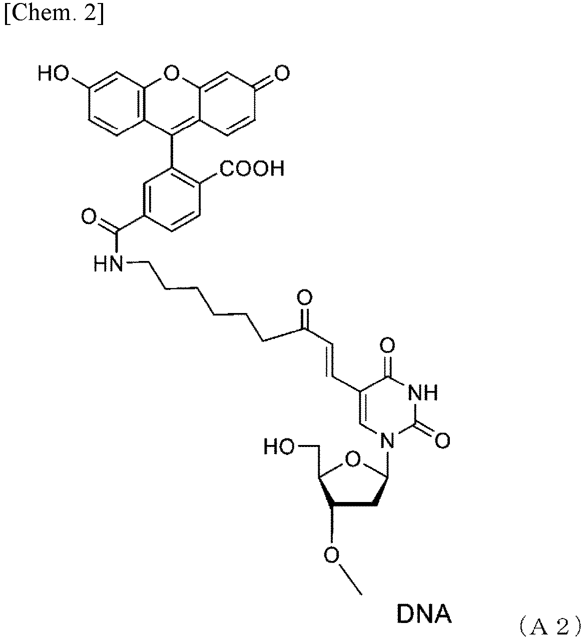

Alternatively, an embodiment of the method according to the first aspect may be, for example, a first embodiment described below. The first embodiment is a method for detecting a target nucleic acid. The method according to the first embodiment is a method in which the substrate described above in the first aspect is applied to the ICA reaction.

### (First Embodiment)

A method of the first embodiment includes the steps of: (a) forming a first cleavage structure composed of a target nucleic acid, a first oligonucleotide and a second oligonucleotide; (b) cleaving the first oligonucleotide constituting the first cleavage structure by a cleavage agent having 5' nuclease activity to produce a third oligonucleotide composed of a 5' side portion derived from the first oligonucleotide; (c) forming a second α-cleavage structure composed of the third oligonucleotide, a fourth oligonucleotide and a detection nucleic acid; (d) cleaving the fourth oligonucleotide constituting the second α-cleavage structure by a cleavage agent having 5' nuclease activity to produce a fifth oligonucleotide composed of a 5' side portion derived from the fourth oligonucleotide; and (e) detecting the fifth oligonucleotide.

The steps (a) to (e) will be described in detail with reference to the drawings. In the following steps, the first oligonucleotide corresponds to the above-mentioned allele probe, and the second oligonucleotide corresponds to the above-mentioned invader oligo. The detection nucleic acid corresponds to the above-mentioned FRET cassette.

### <<Step (a)>>

As illustrated in Fig. 3, in step (a), a first cleavage structure is formed of a target nucleic acid 1, a first oligonucleotide and a second oligonucleotide.

The target nucleic acid 1 includes a first region (hereinafter, also referred to as a T1 region), a second region (hereinafter, also referred to as a T2 region) and a third region (hereinafter, also referred to as a T3 region). The T1 region is adjacent to and downstream of the T2 region, and the T2 region is adjacent to and downstream of the T3 region.

The first oligonucleotide has a nucleotide sequence completely complementary to the T3 region on the 3' side portion, and the second oligonucleotide has a nucleotide sequence completely complementary to the T2 region on the 3' side portion and a nucleotide sequence completely complementary to the T1 region on the 5' side portion.

That is, the first oligonucleotide is an oligonucleotide capable of hybridizing to the T3 region of the target nucleic acid, and the second oligonucleotide is an oligonucleotide capable of hybridizing to the T1 and T2 regions of the target nucleic acid. Accordingly, by adding and mixing the target nucleic acid, the first oligonucleotide and the second oligonucleotide in the reaction solution, a first cleavage structure can be obtained in which the 5' side portion of the region of the first oligonucleotide annealed to the T3 region overlaps one or more nucleotide sequences in the 3' side portion of the second oligonucleotide annealed to the T2 region when the second oligonucleotide is annealed to the T1 and T2 regions and the first oligonucleotide is annealed to the T3 region.

Fig. 3 is a diagram schematically illustrating a first cleavage structure. In Fig. 3, "1" indicates the target nucleic acid, "T1" to "T3" indicate the T1 region to the T3 region, "n1" indicates the first oligonucleotide, and "n2" indicates the second oligonucleotide. Thus, the first cleavage structure has a branched structure in which the second oligonucleotide invades under the first oligonucleotide, and the 5' side of the first oligonucleotide overlaps the second oligonucleotide.

### <<Step (b)>>

The first oligonucleotide constituting the first cleavage structure produced in step (a) is cleaved by a cleavage agent having 5' nuclease activity to produce a third oligonucleotide composed of the 5' side portion derived from the first oligonucleotide.

The cleavage agent having 5' nuclease activity recognizes and cleaves the branched structure in the first cleavage structure, that is, the boundary between the 3' side portion annealed to the T3 region and the 5' side portion overlapping the second oligonucleotide in the first oligonucleotide (indicated by the arrowhead in Fig. 3). The oligonucleotide produced by this cleavage, which is composed of the 5' side portion of the region of the first oligonucleotide annealed to the T3 region, is referred to as a third oligonucleotide.

In step (b), after the cleavage reaction, when the 3' side portion annealed to the T3 region of the first oligonucleotide dissociates from the target nucleic acid, an uncleaved first oligonucleotide is again annealed to the T3 region to form a first cleavage structure. Accordingly, the invader reaction is repeated without the need for operations such as thermal denaturation, and even a minute amount of target nucleic acid can be detected with high sensitivity.

The first oligonucleotide and the second oligonucleotide can be designed and synthesized by conventional methods taking into consideration the nucleotide sequence of the target nucleic acid, the type of cleavage agent used, the reaction temperature in the invader reaction, the type of reaction solution, and the like. If the Tm value of the second oligonucleotide is sufficiently higher than the reaction temperature, hybridization between the second oligonucleotide and the target nucleic acid may become too strong. On the other hand, if the Tm value of the second oligonucleotide is too lower than the reaction temperature, annealing efficiency to the target nucleic acid becomes too low. Therefore, the Tm value of the second oligonucleotide is preferably about the reaction temperature.

Further, in order for the invader reaction to be efficiently repeated, the 3' side portion of the first oligonucleotide needs to dissociate from the T3 region promptly after the cleavage reaction. Therefore, the Tm value of the region that hybridizes to the T3 region of the first oligonucleotide is preferably sufficiently lower than the reaction temperature of the invader reaction.

Further, in the second oligonucleotide, the 3' end of the nucleotide sequence completely complementary to the T2 region is preferably the second nucleotide from the 3' end of the second oligonucleotide. Accordingly, the 3' end of the second oligonucleotide is overlapped with the 5' end nucleotide of the site of the first oligonucleotide annealed to the T3 region, making it possible to efficiently obtain a first cleavage structure. In this case, the 3' end nucleotide of the second oligonucleotide may be a nucleotide complementary or non-complementary to the corresponding nucleotide of the target nucleic acid, but is preferably a non-complementary nucleotide. This is because the branched structure in the first cleavage structure is more unstable, which further improves the efficiency of the invader reaction.

When the target nucleic acid contains a specific nucleotide (target base) in a nucleotide sequence such as a genetic polymorphism, the target base is preferably located near the T2 region or the T3 region in the target nucleic acid, more preferably near the branched structure in the first cleavage structure, and still more preferably in the T2 region near the branched structure. By designing the first oligonucleotide and the second oligonucleotide so that the target base is located near the branched structure, the accuracy in detecting the target base can be improved.

The cleavage agent having 5' nuclease activity used in step (b) is not particularly limited as long as it can recognize the branched structure and has 5' nuclease activity, but is preferably a structure-specific enzyme. Further, it may be an enzyme having only 5' nuclease activity, or may be an enzyme having other enzyme activity. Furthermore, the structure-specific enzyme may be an enzyme derived from any organism species, or may be a synthetic enzyme.

The cleavage agent is preferably an enzyme having 5' nuclease activity, more preferably a modified DNA polymerase having 5' nuclease activity but lacking polymerase activity, and still more preferably a flap endonuclease. The flap endonuclease is the same as that described above.

The cleavage agent having 5' nuclease activity is preferably a thermostable enzyme having 5' nuclease activity. By using a thermostable enzyme, PCR and the invader reaction can be performed using the same reaction system when the target nucleic acid is amplified by PCR in advance.

The amounts of the first oligonucleotide and the second oligonucleotide added to the reaction solution are not particularly limited, but it is preferred to add more of the first oligonucleotide than the second oligonucleotide. The reason for this is that, when the amount of the first oligonucleotide added is higher, the invader reaction is expected to be repeated more efficiently. For example, the added amount of the first oligonucleotide is preferably twice or greater than the added amount of the second oligonucleotide, more preferably approximately 2 times to 100 times, still more preferably approximately 5 times to 50 times, and particularly preferably approximately 10 times.

The composition of the reaction solution is not particularly limited as long as it can form a cleavage structure and does not inhibit 5' nuclease activity of the cleavage agent used, and can be appropriately determined taking into consideration the type of the target nucleic acid, the types of the first oligonucleotide and the second oligonucleotide, the type of the cleavage agent used, and the like. In addition, the reaction temperature can also be appropriately determined taking into consideration the type of the cleavage agent used, the Tm values of the first oligonucleotide and the second oligonucleotide, and the like.

### <<Step (c)>>

As illustrated in Fig. 4, in step (c), a second α-cleavage structure is formed of the third oligonucleotide produced in step (b), a fourth oligonucleotide and a detection nucleic acid 2.

The detection nucleic acid includes a first region (hereinafter, also referred to as a D1 region), a second region (hereinafter, also referred to as a D2 region) and a third region (hereinafter, also referred to as a D3 region), the D1 region being adjacent to and downstream of the D2 region, the D2 region being adjacent to and downstream of the D3 region. Further, the fourth oligonucleotide has a nucleotide sequence completely complementary to the third region of the detection nucleic acid on the 3' side portion, and the third oligonucleotide has a nucleotide sequence completely complementary to the second region of the detection nucleic acid on the 3' side portion and a nucleotide sequence completely complementary to the first region of the detection nucleic acid on the 5' side portion.

That is, the fourth oligonucleotide is an oligonucleotide capable of hybridizing to the D3 region of the detection nucleic acid, and the third oligonucleotide is an oligonucleotide capable of hybridizing to the D1 and D2 regions of the detection nucleic acid. Accordingly, by adding and mixing the detection nucleic acid, the third oligonucleotide and the fourth oligonucleotide in the reaction solution, a second α-cleavage structure can be obtained in which the 5' side portion of the region of the fourth oligonucleotide annealed to the D3 region overlaps one or more nucleotide sequences in the 3' side portion of the third oligonucleotide annealed to the D2 region when the third oligonucleotide is annealed to the D1 and D2 regions and the fourth oligonucleotide is annealed to the D3 region.

Fig. 4 is a diagram schematically illustrating a second α-cleavage structure. In Fig. 4, "2" indicates the detection nucleic acid, "D1" to "D3" indicate the D1 region to the D3 region, "n3" indicates the third oligonucleotide, and "n4" indicates the fourth oligonucleotide. Thus, the second α-cleavage structure has a branched structure in which the third oligonucleotide invades under the fourth oligonucleotide, and the 5' side of the fourth oligonucleotide overlaps the third oligonucleotide, and in the second α-cleavage structure, the fourth oligonucleotide acts as an allele probe and the third oligonucleotide acts as an invader oligo.

### <<Step (d)>>

In step (d), the fourth oligonucleotide constituting the second α-cleavage structure produced in step (c) is cleaved by a cleavage agent having 5' nuclease activity to produce a fifth oligonucleotide composed of the 5' side portion derived from the fourth oligonucleotide.

Specifically, as in step (b), the boundary between the 3' side portion annealed to the D3 region and the 5' side portion overlapping the third oligonucleotide in the fourth oligonucleotide, indicated by the arrowhead in Fig. 4, is cleaved by the cleavage agent having 5' nuclease activity. The oligonucleotide produced by this cleavage, which is composed of the 5' side portion of the region of the fourth oligonucleotide annealed to the D3 region, is referred to as a fifth oligonucleotide.

The fourth oligonucleotide may be the substrate described above in the first aspect. For example, the fourth oligonucleotide may be one that is labeled with a fluorescent molecule that functions as a fluorescent substance and a fluorescent molecule that functions as a quenching substance in the 5' side portion (portion that becomes the fifth oligonucleotide) and the site annealed to the D3 region, respectively.

That is, in the fourth oligonucleotide, the fluorescent substance is in spatial proximity to the quenching substance, and fluorescence emission from the fluorescent substance is suppressed by the quenching substance. The peak wavelength of the absorption spectrum of the quenching substance is on the shorter wavelength side of the peak wavelength of the emission spectrum of the fluorescent substance.

When the fourth oligonucleotide is labeled with a fluorescent substance and a quenching substance, the number of bases between the nucleic acid to which the fluorescent substance is bound and the nucleic acid to which the quenching substance is bound is preferably 11 bases or greater and 30 base or less, more preferably 4 bases or greater and 10 bases or less, and still more preferably 1 base or greater and 3 bases or less.

Due to the fluorescent substance of the fourth oligonucleotide being bound to a portion other than a base, a triplex structure can be efficiently formed, improving the reactivity in ICA. The term "fluorescent substance being bound to a portion other than a base" means that the fluorescent substance is bound to a sugar residue or a phosphate group, among sugar residues, phosphate groups and bases of the oligonucleotide constituting the fluorescent substrate. In particular, it is preferred that the fluorescent substance is bound to a phosphate group of the fluorescent substrate.

In the fourth oligonucleotide (also referred to as a fluorescent substrate), a phosphate group is preferably added to the 5' end, and the fluorescent substance is preferably bound to the phosphate group added to the 5' end.

As described later in the examples, a fluorescent substrate in which a phosphate group is added to the 5' end and a fluorescent substance is bound to the phosphate group added to the 5' end has improved reactivity in ICA and thus efficiently forms a triplex structure compared to a fluorescent substrate in which a fluorescent substance is bound to a base.

The above formula (A1) is a chemical formula showing an example fluorescent substrate in which a phosphate group is added to the 5' end and a fluorescent substance is bound to the phosphate group added to the 5' end. As shown in the above formula (A1), the fluorescent substance may be bound to the phosphate group added to the 5' end via a linker containing a hydrocarbon group having approximately 1 to 100 carbon atoms.

The linker preferably has approximately 1 to 30 carbon atoms, and more preferably approximately 1 to 10 carbon atoms. A linker having the number of carbon atoms equal to or greater than the lower limit can maintain an appropriate distance between the fluorescent substance and the site cleaved by the cleavage agent having 5' nuclease activity. As a result, a cleavage reaction by the cleavage agent is less likely to be inhibited by the fluorescent substance, improving the reaction efficiency by the cleavage agent. A linker having the number of carbon atoms equal to or smaller than the upper limit can reduce the distance between the fluorescent substance and the quenching substance. As a result, the quenching effect of the quenching substance is improved.

The cleavage reaction in step (d) separates the fluorescent substance from the quenching substance in the fourth oligonucleotide, and fluorescence is emitted from the fluorescent substance.

### <<Step (e)>>

In step (e), the fluorescence emitted from the fluorescent substance of the fifth oligonucleotide produced in step (d) is detected. The fluorescence may be observed with a fluorescence microscope.

Alternatively, an embodiment of the method according to the first aspect may be, for example, a second embodiment described below. The second embodiment is a method for detecting a target nucleic acid. The method according to the second embodiment is a method in which the substrate described above in the first aspect is applied to the ICA reaction.

### (Second Embodiment)

The second embodiment includes the following steps (f) to (h) instead of the steps (c) to (e), after the above-mentioned steps (a) and (b).

In the following steps, the first oligonucleotide corresponds to the above-mentioned allele probe, and the second oligonucleotide corresponds to the above-mentioned invader oligo. A sixth oligonucleotide corresponds to the above-mentioned FRET cassette.

### <<Step (f)>>

As illustrated in Fig. 5, in step (f), a second β-cleavage structure is formed of the third oligonucleotide produced in step (b) and a sixth oligonucleotide.

The sixth oligonucleotide includes a first region (hereinafter, also referred to as a P6-1 region), a second region (hereinafter, also referred to as a P6-2 region) and a third region (hereinafter, also referred to as a P6-3 region), the P6-1 region being adjacent to and downstream of the P6-2 region, the P6-2 region being adjacent to and downstream of the P6-3 region, and the P6-1 region has a nucleotide sequence completely complementary to the third oligonucleotide and the P6-2 region has a nucleotide sequence capable of forming a intermolecular hairpin structure.

Fig. 5 is a diagram schematically illustrating a second β-cleavage structure. In Fig. 5, "n3" indicates the third oligonucleotide, "n6" indicates the sixth oligonucleotide, and "P6-1" to "P6-3" indicate the P6-1 region to the P6-3 region. Thus, the second β-cleavage structure has a branched structure in which the third oligonucleotide invades under the sixth oligonucleotide, and the P6-3 region of the sixth oligonucleotide overlaps the third oligonucleotide, and in the second β-cleavage structure, the sixth oligonucleotide acts as an allele probe and the third oligonucleotide acts as an invader oligo.

### <<Step (g)>>

In step (g), the sixth oligonucleotide constituting the second β-cleavage structure produced in step (f) is cleaved by a cleavage agent having 5' nuclease activity to produce a seventh oligonucleotide composed of the 5' side portion derived from the sixth oligonucleotide (that is, P6-3 region).

Specifically, as in step (b), the boundary between the P6-2 region and the P6-3 region of the 5' side portion overlapping the third oligonucleotide in the sixth oligonucleotide, indicated by the arrowhead in Fig. 5, is cleaved by the cleavage agent having 5' nuclease activity. The oligonucleotide produced by this cleavage, which is composed of the 5' side portion of the P6-2 region of the sixth oligonucleotide is referred to as a seventh oligonucleotide.

The sixth oligonucleotide may be the substrate described above in the first aspect. For example, the sixth oligonucleotide may be one that is labeled with a fluorescent molecule that functions as a fluorescent substance and a fluorescent molecule that functions as a quenching substance (Q) in the P6-3 region (portion that becomes the seventh oligonucleotide) and the P6-2 region, respectively.

That is, in the sixth oligonucleotide, the fluorescent substance is in spatial proximity to the quenching substance, and fluorescence emission from the fluorescent substance is suppressed by the quenching substance. The peak wavelength of the absorption spectrum of the quenching substance is on the shorter wavelength side of the peak wavelength of the emission spectrum of the fluorescent substance.

The sixth oligonucleotide of the present embodiment preferably has a length of approximately 20 bases to 150 bases. Further, the number of base pairs constituting the hairpin structure is preferably approximately 5 to 50.

When the sixth oligonucleotide is labeled with a fluorescent substance and a quenching substance, the number of bases between the nucleic acid to which the fluorescent substance is bound and the nucleic acid to which the quenching substance is bound is preferably 11 bases or greater and 30 base or less, more preferably 4 bases or greater and 10 bases or less, and still more preferably 1 base or greater and 3 bases or less.

Due to the fluorescent substance of the sixth oligonucleotide being bound to a portion other than a base, a triplex structure can be efficiently formed, improving the reactivity in ICA. The term "fluorescent substance being bound to a portion other than a base" means that the fluorescent substance is bound to a sugar residue or a phosphate group, among sugar residues, phosphate groups and bases of the oligonucleotide constituting the fluorescent substrate. In particular, it is preferred that the fluorescent substance is bound to a phosphate group of the fluorescent substrate.

In the sixth oligonucleotide (also referred to as a fluorescent substrate), a phosphate group is preferably added to the 5' end, and the fluorescent substance is preferably bound to the phosphate group added to the 5' end.

As described later in the examples, a fluorescent substrate in which a phosphate group is added to the 5' end and a fluorescent substance is bound to the phosphate group added to the 5' end has improved reactivity in ICA and thus efficiently forms a triplex structure compared to a fluorescent substrate in which a fluorescent substance is bound to a base.

The above formula (A1) is a chemical formula showing an example fluorescent substrate in which a phosphate group is added to the 5' end and a fluorescent substance is bound to the phosphate group added to the 5' end. As shown in the above formula (A1), the fluorescent substance may be bound to the phosphate group added to the 5' end via a linker containing a hydrocarbon group having approximately 1 to 100 carbon atoms.

The linker preferably has approximately 1 to 30 carbon atoms, and more preferably approximately 1 to 10 carbon atoms. A linker having the number of carbon atoms equal to or greater than the lower limit can maintain an appropriate distance between the fluorescent substance and the site cleaved by the cleavage agent having 5' nuclease activity. As a result, a cleavage reaction by the cleavage agent is less likely to be inhibited by the fluorescent substance, improving the reaction efficiency by the cleavage agent. A linker having the number of carbon atoms equal to or smaller than the upper limit can reduce the distance between the fluorescent substance and the quenching substance. As a result, the quenching effect of the quenching substance is improved.

The cleavage reaction in step (g) separates the fluorescent substance from the quenching substance (Q) in the seventh oligonucleotide, and fluorescence is emitted from the fluorescent substance.

### <<Step (h)>>

In step (h), the fluorescence emitted from the fluorescent substance of the seventh oligonucleotide produced in step (g) is detected. The fluorescence may be observed with a fluorescence microscope.

There are no particular limitations on the temperature, pH, salt concentration, buffer solution, and the like of the reaction solution for forming the first cleavage structure, the second α-cleavage structure and the second β-cleavage structure described above, and these can be appropriately set by those skilled in the art. The pH of the reaction solution is preferably 6.5 to 10.0, and more preferably 7.5 to 9.0. The concentration of the salt having a buffer effect is preferably 5 mM to 250 mM, and more preferably 10 mM to 100 mM. The salt having a buffer effect is not particularly limited, and examples thereof include cacodylates, phosphates and tris salts. The reaction solution preferably contains alkali metal salt and/or alkali earth metal salt, and more preferably sodium chloride and/or magnesium chloride.

### <Embodiment B>

### [Single-Stranded Oligonucleotide]

In one embodiment, the present invention provides a single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the fluorescent substance is bound to a portion other than the base.

The single-stranded oligonucleotide of the present embodiment is a fluorescent substrate for ICA, and can also be said to be a substrate for the flap endonuclease. Examples of the flap endonuclease include flap endonuclease 1 (NCBI accession number: WP_011012561.1, Holliday junction 5' flap endonuclease (GEN1) (NCBI accession number: NP_001123481.3), excision repair protein (NCBI accession number: AAC37533.1), and the like.

Here, when a hairpin structure is formed by self-hybridization on the 5' side and a single-stranded oligonucleotide having a complementary base sequence on the 3' side hybridizes, a triplex structure formed on the 5' end portion corresponds to the second triplex structure 160 shown in Fig. 1. That is, a single-stranded oligonucleotide having a complementary base sequence on the 3' side corresponds to the nucleic acid fragment 140 derived from the flap site 140.

As described later in the examples, due to the fluorescent substance of the fluorescent substrate being bound to a portion other than a base, a triplex structure can be efficiently formed, improving the reactivity in ICA. The term "fluorescent substance being bound to a portion other than a base" means that the fluorescent substance is bound to a sugar residue or a phosphate group, among sugar residues, phosphate groups and bases of the oligonucleotide constituting the fluorescent substrate. In particular, it is preferred that the fluorescent substance is bound to a phosphate group of the fluorescent substrate.

In this specification, the term "reactivity in ICA is improved" may mean that the fluorescence signal detected from the result of ICA reaches a predetermined value in a shorter period of time. Alternatively, it may mean that the fluorescence signal intensity detected from the result of ICA reaches a higher value. Alternatively, it may mean that the background fluorescence signal detected from the result of ICA is maintained at a lower value.

The single-stranded oligonucleotide of the present embodiment preferably has a length of approximately 20 bases to 150 bases. Further, the number of base pairs constituting the hairpin structure is preferably approximately 5 to 50.

### (Fluorescent Substance)

The fluorescent substance is not particularly limited, and examples thereof include fluorescein (molecular weight 332.3), ATTO 425 (molecular weight 401.45), Alexa 488 (molecular weight 534.47), ATTO 542 (molecular weight 914), Yakima Y (molecular weight 718.33), Redmond R (molecular weight 445.3), ATTO 643 (molecular weight 836), Alexa 647 (molecular weight 860), Alexa 680 (molecular weight 1050), Alexa 568 (molecular weight 694.7), FAM (molecular weight 332.3), ATTO 633 (molecular weight 652.2), Cy 5 (molecular weight 483.7), HiLyte Fluor 647 (molecular weight 1205.6), ATTO 665 (molecular weight 723), Alexa 594 (molecular weight 722.8), Cy 3 (molecular weight 457.6), ROX (molecular weight 534.6), and the like. The molecular weight of the fluorescent substance is preferably 350 to 1,100, more preferably 445 to 1,100, and still more preferably 445 to 914. As described later in the examples, the molecular weight of the fluorescent substance in the above range tends to provide a high signal noise ratio (S/N ratio), which is preferred.

In the single-stranded oligonucleotide of the fluorescent substrate, a phosphate group is preferably added to the 5' end, and the fluorescent substance is preferably bound to the phosphate group added to the 5' end.

As described later in the examples, a fluorescent substrate in which a phosphate group is added to the 5' end and a fluorescent substance is bound to the phosphate group added to the 5' end has improved reactivity in ICA and thus efficiently forms a triplex structure compared to a fluorescent substrate in which a fluorescent substance is bound to a base.

The following formula (B1) is a chemical formula showing an example fluorescent substrate in which a phosphate group is added to the 5' end and a fluorescent substance is bound to the phosphate group added to the 5' end. As shown in the following formula (B 1), the fluorescent substance may be bound to the phosphate group added to the 5' end via a linker containing a hydrocarbon group having approximately 1 to 100 carbon atoms. The linker preferably has approximately 1 to 30 carbon atoms, and more preferably approximately 1 to 10 carbon atoms. A linker having the number of carbon atoms equal to or greater than the lower limit can maintain an appropriate distance between the fluorescent substance and the site cleaved by the flap endonuclease. As a result, a cleavage reaction by the flap endonuclease is less likely to be inhibited by the fluorescent substance, improving the efficiency of cleavage by the flap endonuclease. Further, a linker having the number of carbon atoms equal to or smaller than the upper limit can reduce the distance between the fluorescent substance and the quenching substance. As a result, the quenching effect of the quenching substance is improved.

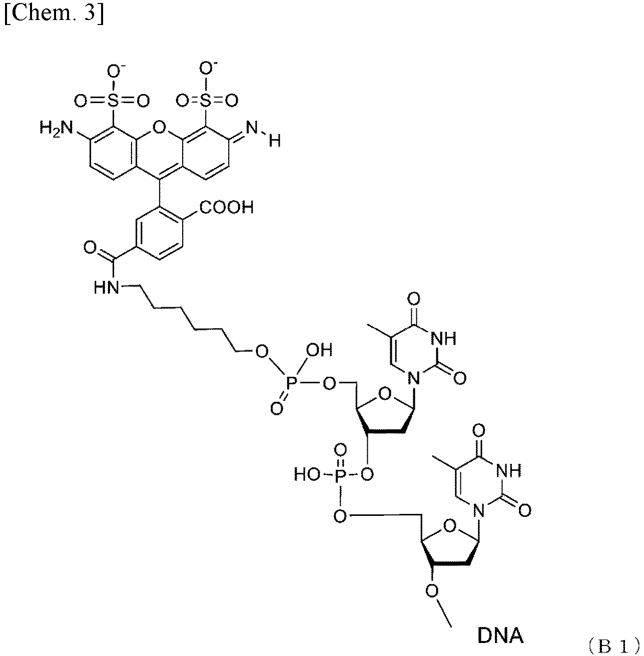

The following formula (B2) is a chemical formula showing an example fluorescent substrate in which a fluorescent substance is bound to a base.

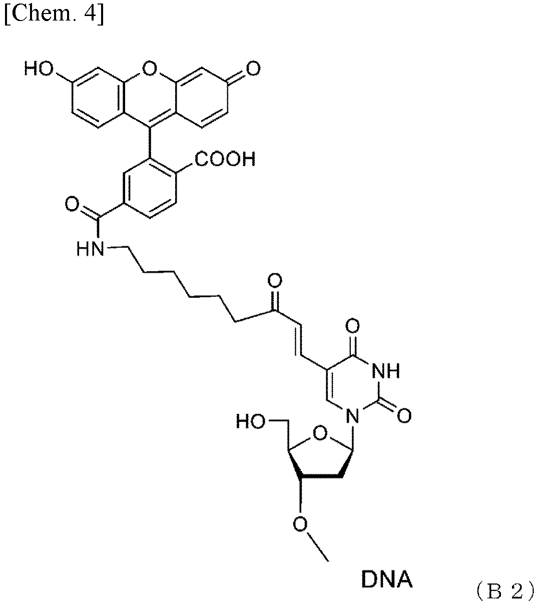

### (Quenching Substance)

The quenching substance is not particularly limited as long as it can quench the fluorescence of the fluorescent substance used, and examples thereof include Black Hole Quencher (BHQ) (registered trademark)-1, BHQ (registered trademark)-2, BHQ (registered trademark)-3, Tide Quencher 1 (TQ1), Tide Quencher 2 (TQ2), Tide Quencher 2WS (TQ2WS), Tide Quencher 3 (TQ3), Tide Quencher 3WS (TQ3WS), Tide Quencher 4 (TQ4), Tide Quencher 4WS (TQ4WS), Tide Quencher 5 (TQ5), Tide Quencher 5WS (TQ5WS), Tide Quencher 6WS (TQ6WS), Tide Quencher 7WS (TQ7WS), QSY35, QSY7, QSY9, QSY21, Iowa Black FQ and Iowa Black RQ. The quenching substance is selected that can quench the fluorescence of the fluorescent substance used.

When a fluorescent substance is in spatial proximity to a quenching substance, fluorescence from the fluorescent substance is quenched by the quenching substance. The term "fluorescence is quenched" means as follows. In the absence of the quenching substance, the intensity of fluorescence emitted from the fluorescent substance when the fluorescent substance is irradiated with excitation light is defined as A. Further, in the presence of the quenching substance in spatial proximity to the fluorescent substance, the intensity of fluorescence emitted from the fluorescent substance when the fluorescent substance is irradiated with excitation light is defined as B. Here, the "fluorescence is quenched" means that the value of B/A is 40% or less.

When the fluorescent substance is in spatial proximity to the quenching substance, the distance between the fluorescent substance and the quenching substance is not particularly limited as long as the fluorescence emitted from the fluorescent substance is suppressed by the quenching substance, and is preferably 10 nm or less, more preferably 5 nm or less, and still more preferably 2 nm or less.

In the fluorescent substrate of the present embodiment, the quenching substance is preferably bound to the base of the nucleotide residue located on the 3' side of the fluorescent substance.

As described later in the examples, a fluorescent substrate in which a quenching substance is located on the 3' side of a fluorescent substance tends to have higher reactivity in ICA than a fluorescent substrate in which a quenching substance is located on the 5' side of a fluorescent substance. That is, an aspect in which a quenching substance is labeled on the 3' side of a fluorescent substance and an oligonucleotide fragment containing a fluorescent substance is released when cleaved by a flap endonuclease tends to have higher reactivity in ICA than an aspect in which a quenching substance is released from a fluorescent substrate.

The quenching substance can be incorporated into the sugar-phosphate backbone. The following formula (B3) is a chemical formula showing an example of the quenching substance incorporated into the sugar-phosphate backbone of a nucleotide. The quenching substance in the following formula (B3) is BHQ (registered trademark)-1.

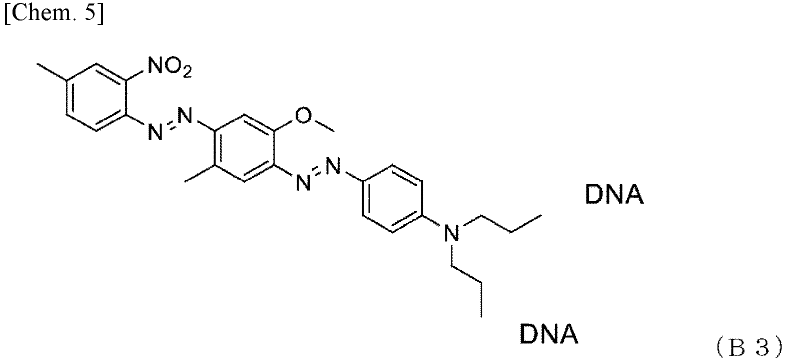

However, as described later in the examples, a fluorescent substrate in which a quenching substance is bound to the base tends to have higher reactivity in ICA than a fluorescent substrate in which a quenching substance is incorporated into the sugar-phosphate backbone.

The following formula (B4) is a chemical formula showing an example fluorescent substrate in which a quenching substance is bound to a base. The quenching substance in the following formula (B4) is BHQ (registered trademark)-1.

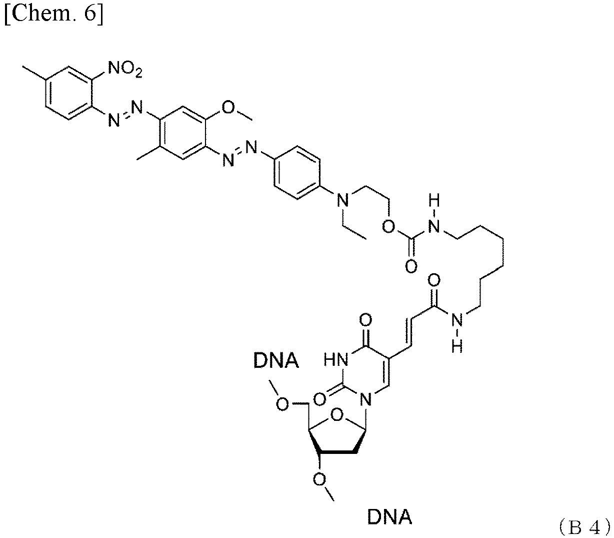

### (Oligonucleotide)

The single-stranded oligonucleotide constituting the fluorescent substrate of the present embodiment is not particularly limited as long as the advantageous effects of the present invention are achieved, and may be a natural nucleic acid or a synthetic nucleic acid.

Examples of the natural nucleic acids include genomic DNA, mRNA, rRNA, hnRNA, miRNA and tRNA. The natural nucleic acids may be those recovered from living organisms or may be those recovered from water, organic matter, or the like that has been in contact with living organisms. Methods for recovering natural nucleic acids include known methods such as the phenol/chloroform method.

Examples of the synthetic nucleic acids include synthetic DNA, synthetic RNA, cDNA, bridged nucleic acid (BNA) and locked nucleic acid (LNA).

Methods for synthesizing the nucleic acid are not particularly limited, and examples thereof include known chemical synthesis methods such as the DNA solid-phase synthesis by phosphoramidite method, known nucleic acid amplification methods and reverse transcription reactions. Examples of the nucleic acid amplification reaction include PCR, LAMP, SMAP, NASBA and RCA.

In the fluorescent substrate of the present embodiment, it is preferred that the fluorescent substance is located in proximity to a 5' end nucleotide residue, and the base of the 5' end nucleotide residue is a pyrimidine base. A purine base has a tendency to quench the fluorescence of the fluorescent substance. Therefore, when the base of the 5' end nucleotide residue is a pyrimidine base, quenching of the fluorescence of the fluorescent substance is suppressed, which is preferred.

In the fluorescent substrate of the present embodiment, it is preferred that both the fluorescent substance and the quenching substance are present in one strand of the double-stranded oligonucleotide portion of the hairpin structure.

As described later in the examples, the reactivity in ICA tends to be higher in a fluorescent substrate in which both a fluorescent substance and a quenching substance are present in one strand of the double-stranded oligonucleotide portion of the hairpin structure than in a fluorescent substrate in which a fluorescent substance is present in one strand of the double-stranded oligonucleotide portion of the hairpin structure and a quenching substance is present in the other strand. More specifically, in a fluorescent substrate in which a fluorescent substance is present in one strand of the double-stranded oligonucleotide portion of the hairpin structure and a quenching substance is present in the other strand, the background fluorescence signal may increase.

### [Method for Improving Efficiency of Forming Triplex Structure in Fluorescent Substrate for ICA]

In one embodiment, the present invention provides a method for improving the efficiency of forming a triplex structure in a fluorescent substrate for ICA, the method including a step of adding a fluorescent substrate in which a fluorescent substance is bound to a portion other than the base to a reaction solution for ICA.

The fluorescent substrate for ICA is a single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognize the triplex structure, whereby the fluorescent substance is released from the quenching substance, the fluorescent substance emitting fluorescence when irradiated with excitation light.

The method of the present embodiment is a method for improving the efficiency of forming a triplex structure in a fluorescent substrate for flap endonuclease, and can be said to be a method including the steps of: preparing a first single-stranded oligonucleotide labeled with a fluorescent substance and a quenching substance, and a second single-stranded oligonucleotide having a complementary base sequence on the 3' side of the first single-stranded oligonucleotide; and bringing the first single-stranded oligonucleotide into contact with the second single-stranded oligonucleotide, wherein the first single-stranded oligonucleotide forms a hairpin structure by self-hybridization on the 5' side, and when the second single-stranded oligonucleotide hybridizes, a triplex structure is formed on the 5' end portion, and cleaved by the flap endonuclease, whereby the fluorescent substance is released from the quenching substance, the fluorescent substance emitting fluorescence when irradiated with excitation light, and the fluorescent substance is bound to a portion other than the base of the first single-stranded oligonucleotide.

**In** the method of the present embodiment, the ICA, fluorescent substance, quenching substance, oligonucleotide, and the like are the same as those described above.

In the method of the present embodiment, it is preferred that, in the fluorescent substrate, a phosphate group is added to the 5' end, and the fluorescent substance is bound to the phosphate group added to the 5' end.

In the method of the present embodiment, it is preferred that, in the fluorescent substrate, the quenching substance is bound to the base of the nucleotide residue located on the 3' side of the fluorescent substance.

In the method of the present embodiment, it is preferred that, in the fluorescent substrate, the base of the 5' end nucleotide residue is a pyrimidine base.

In the method of the present embodiment, it is preferred that, in the fluorescent substrate, the quenching substance is labeled on the 3' side of the fluorescent substance, and an oligonucleotide fragment containing the fluorescent substance is released when cleaved by a flap endonuclease.

In the method of the present embodiment, the molecular weight of the fluorescent substrate is preferably 350 to 1,100, more preferably 445 to 1,100, and still more preferably 445 to 914. As described later in the examples, the molecular weight of the fluorescent substance in the above range tends to provide a high signal noise ratio (S/N ratio), which is preferred.

In the method of the present embodiment, it is preferred that, in the fluorescent substrate, both the fluorescent substance and the quenching substance are present in one strand of the double-stranded oligonucleotide portion of the hairpin structure.

In the method of the present embodiment, ICA is preferably performed in a microspace. Specifically, it is preferred to perform ICA using a device including a well array having a plurality of microwells.

The wells may be used without treatment, or may be pre-treated such as by immobilizing an extraction reagent, a detection reagent such as antibody, or the like on the inner wall of the wells, or covering the well openings with a lipid bilayer depending on the purpose.

The device may have flow channels, and a reaction solution for ICA may be supplied to the wells of the well array through the flow channels.

The well may have a diameter of approximately 3 µm and a depth of approximately 4.5 µm, for example. The wells may be arranged on the substrate member to form a well array arranged in a triangular lattice or a square lattice.

Examples of the material for the substrate member include cycloolefin polymer, cycloolefin copolymer, silicone, polypropylene, polycarbonate, polystyrene, polyethylene, polyvinyl acetate, fluororesin and amorphous fluororesin.

The number of wells in the well array is preferably approximately 100,000 to 6,000,000 per device. The volume per well is preferably 1 fL to 6 pL.

### <Embodiment C>

### [Single-Stranded Oligonucleotide]

In one embodiment, the present invention provides a single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and the single-stranded oligonucleotide includes a non-complementary base pair within the hairpin structure.

The single-stranded oligonucleotide of the present embodiment is a fluorescent substrate for ICA, and can also be said to be a substrate for the flap endonuclease. Examples of the flap endonuclease include flap endonuclease 1 (NCBI accession number: WP_011012561.1, Holliday junction 5' flap endonuclease (GEN1) (NCBI accession number: NP_001123481.3), excision repair protein (NCBI accession number: AAC37533.1), and the like.

Here, when a hairpin structure is formed by self-hybridization on the 5' side and a single-stranded oligonucleotide having a complementary base sequence on the 3' side hybridizes, a triplex structure formed on the 5' end portion corresponds to the second triplex structure 160 shown in Fig. 1. That is, a single-stranded oligonucleotide having a complementary base sequence on the 3' side corresponds to the nucleic acid fragment 140 derived from the flap site 140.

As described later in the examples, an increase in background in ICA can be suppressed by including a non-complementary base pair within the hairpin structure.

In the single-stranded oligonucleotide of the present embodiment, the number of non-complementary base pairs may be 1 to 3, preferably 1 or 2, and more preferably 1.

In this specification, the background in ICA may refer to, for example, a fluorescence signal detected when ICA is performed in the absence of a target nucleic acid. The term "an increase in background in ICA is suppressed" means that the background fluorescence signal detected as a result of ICA is maintained at a lower value.

The single-stranded oligonucleotide of the present embodiment preferably has a length of approximately 20 bases to 150 bases. Further, the number of base pairs constituting the hairpin structure is preferably approximately 5 to 50.

### (Fluorescent Substance)

The fluorescent substance is not particularly limited, and examples thereof include fluorescein, ATTO 425, Alexa 488, ATTO 542, Yakima Y, Redmond R, ATTO 643, Alexa 647, Alexa 680, Alexa 568, FAM, ATTO 633, Cy 5, HiLyte Fluor 647, ATTO 663, and the like.

In the single-stranded oligonucleotide of the present embodiment, it is preferred that the fluorescent substance is labeled at a 5' end nucleotide residue, and the quenching substance is labeled at a nucleotide residue located closer to the 5' side among nucleotide residues constituting the non-complementary base pair.

As described later in the examples, background can be significantly suppressed by performing ICA using such a fluorescent substrate.

### (Quenching Substance)

The quenching substance is not particularly limited as long as it can quench the fluorescence of the fluorescent substance used, and examples thereof include Black Hole Quencher (BHQ) (registered trademark)-1, BHQ (registered trademark)-2, BHQ (registered trademark)-3, Tide Quencher 1 (TQ1), Tide Quencher 2 (TQ2), Tide Quencher 2WS (TQ2WS), Tide Quencher 3 (TQ3), Tide Quencher 3WS (TQ3WS), Tide Quencher 4 (TQ4), Tide Quencher 4WS (TQ4WS), Tide Quencher 5 (TQ5), Tide Quencher 5WS (TQ5WS), Tide Quencher 6WS (TQ6WS), Tide Quencher 7WS (TQ7WS), QSY35, QSY7, QSY9, QSY21, Iowa Black FQ and Iowa Black RQ. The quenching substance is selected that can quench the fluorescence of the fluorescent substance used.

When a fluorescent substance is in spatial proximity to a quenching substance, fluorescence from the fluorescent substance is quenched by the quenching substance. The term "fluorescence is quenched" means as follows. In the absence of the quenching substance, the intensity of fluorescence emitted from the fluorescent substance when the fluorescent substance is irradiated with excitation light is defined as A. Further, in the presence of the quenching substance in spatial proximity to the fluorescent substance, the intensity of fluorescence emitted from the fluorescent substance when the fluorescent substance is irradiated with excitation light is defined as B. Here, the "fluorescence is quenched" means that the value of B/A is 40% or less.

When the fluorescent substance is in spatial proximity to the quenching substance, the distance between the fluorescent substance and the quenching substance is not particularly limited as long as the fluorescence emitted from the fluorescent substance is suppressed by the quenching substance, and is preferably 10 nm or less, more preferably 5 nm or less, and still more preferably 2 nm or less.

### (Oligonucleotide)

The single-stranded oligonucleotide constituting the fluorescent substrate of the present embodiment is not particularly limited as long as the advantageous effects of the present invention are achieved, and may be a natural nucleic acid or a synthetic nucleic acid.

Examples of the natural nucleic acids include genomic DNA, mRNA, rRNA, hnRNA, miRNA and tRNA. The natural nucleic acids may be those recovered from living organisms or may be those recovered from water, organic matter, or the like that has been in contact with living organisms. Methods for recovering natural nucleic acids include known methods such as the phenol/chloroform method.

Examples of the synthetic nucleic acids include synthetic DNA, synthetic RNA, cDNA, bridged nucleic acid (BNA) and locked nucleic acid (LNA).

Methods for synthesizing the nucleic acid are not particularly limited, and examples thereof include known chemical synthesis methods such as the DNA solid-phase synthesis by phosphoramidite method, known nucleic acid amplification methods and reverse transcription reactions. Examples of the nucleic acid amplification reaction include PCR, LAMP, SMAP, NASBA and RCA.

In the fluorescent substrate of the present embodiment, it is preferred that the fluorescent substance is located in proximity to a 5' end nucleotide residue, and the base of the 5' end nucleotide residue is a pyrimidine base. A purine base has a tendency to quench the fluorescence of the fluorescent substance. Therefore, when the base of the 5' end nucleotide residue is a pyrimidine base, quenching of the fluorescence of the fluorescent substance is suppressed, which is preferred.

### [Method for Suppressing Increase in Background in ICA]

In one embodiment, the present invention provides a method for suppressing an increase in background in ICA, the method including a step of adding a fluorescent substrate containing a non-complementary base pair within the hairpin structure to a reaction solution for ICA.

The fluorescent substrate for ICA is a single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognize the triplex structure, whereby the fluorescent substance is released from the quenching substance, the fluorescent substance emitting fluorescence when irradiated with excitation light.

In the method of the present embodiment, the ICA, fluorescent substance, quenching substance, oligonucleotide, and the like are the same as those described above.

In the method of the present embodiment, it is preferred that, in the fluorescent substrate, the fluorescent substance is labeled at a 5' end nucleotide residue, and the quenching substance is labeled at a nucleotide residue located closer to the 5' side among nucleotide residues constituting the non-complementary base pair.

In the method of the present embodiment, it is preferred that, in the fluorescent substrate, the fluorescent substance is located in proximity to a 5' end nucleotide residue, and the base of the 5' end nucleotide residue is a pyrimidine base.

In the method of the present embodiment, it is preferred that, in the fluorescent substrate, the quenching substance is labeled on the 3' side of the fluorescent substance, and an oligonucleotide fragment containing the fluorescent substance is released when cleaved by a flap endonuclease.

In the method of the present embodiment, ICA is preferably performed in a microspace. Specifically, it is preferred to perform ICA using a device including a well array having a plurality of microwells.

The wells may be used without treatment, or may be pre-treated such as by immobilizing an extraction reagent, a detection reagent such as antibody, or the like on the inner wall of the wells, or covering the well openings with a lipid bilayer depending on the purpose.

The device may have flow channels, and a reaction solution for ICA may be supplied to the wells of the well array through the flow channels.

The well may have a diameter of approximately 3 µm and a depth of approximately 4.5 µm, for example. The wells may be arranged on the substrate member to form a well array arranged in a triangular lattice or a square lattice.

Examples of the material for the substrate member include cycloolefin polymer, cycloolefin copolymer, silicone, polypropylene, polycarbonate, polystyrene, polyethylene, polyvinyl acetate, fluororesin and amorphous fluororesin.

The number of wells in the well array is preferably approximately 100,000 to 6,000,000 per device. The volume per well is preferably 1 fL to 6 pL.

### <Embodiment D>

### [Single-Stranded Oligonucleotide]

In one embodiment, the present invention provides a single-stranded oligonucleotide labeled with a fluorescent substance and a quenching substance, wherein a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and a nucleotide residue labeled with the fluorescent substance and a nucleotide residue labeled with the quenching substance are separated from each other by 4 or more residues.

The single-stranded oligonucleotide of the present embodiment is a fluorescent substrate for ICA, and can also be said to be a substrate for the flap endonuclease. Examples of the flap endonuclease include flap endonuclease 1 (NCBI accession number: WP_011012561.1, Holliday junction 5' flap endonuclease (GEN1) (NCBI accession number: NP_001123481.3), excision repair protein (NCBI accession number: AAC37533.1), and the like.

Here, when a hairpin structure is formed by self-hybridization on the 5' side and a single-stranded oligonucleotide having a complementary base sequence on the 3' side hybridizes, a triplex structure formed on the 5' end portion corresponds to the second triplex structure 160 shown in Fig. 1. That is, a single-stranded oligonucleotide having a complementary base sequence on the 3' side corresponds to the nucleic acid fragment 140 derived from the flap site 140.

As described later in the examples, the efficiency of cleavage by the flap endonuclease in ICA can be improved by separating the nucleotide residue labeled with a fluorescent substance and the nucleotide residue labeled with a quenching substance from each other by 4 or more residues in the fluorescent substrate. As a result, a fluorescence signal detected when ICA is performed can be enhanced.

The term "efficiency of cleavage by the flap endonuclease in ICA is improved" means that a fluorescence signal detected when ICA is performed is higher than that when ICA is performed using a fluorescent substrate in which the distance between the nucleotide residue labeled with a fluorescent substance and the nucleotide residue labeled with a quenching substance is less than 4 residues.

The single-stranded oligonucleotide of the present embodiment can also be said to be a single-stranded oligonucleotide with improved efficiency of cleavage by a flap endonuclease.

The term "separating the nucleotide residue labeled with a fluorescent substance and the nucleotide residue labeled with a quenching substance from each other by 4 or more residues" means that the distance between the nucleotide residue labeled with a fluorescent substance and the nucleotide residue labeled with a quenching substance is 4 or more nucleotide residues.

For example, when a fluorescent substance is labeled at the first nucleotide residue and a quenching substance is labeled at the fifth nucleotide residue, the distance between the nucleotide residue labeled with the fluorescent substance and the nucleotide residue labeled with the quenching substance is 4 residues.

In this specification, the term "distance is 4 residues" can be rephrased as "distance is 4 bases long," "distance is 4 bases," "the number of bases is four," and the like.

As described later in the examples, the distance between the nucleotide residue labeled with a fluorescent substance and the nucleotide residue labeled with a quenching substance is preferably 4 or 5 residues, and more preferably 4 residues.

The single-stranded oligonucleotide of the present embodiment preferably has a length of approximately 20 bases to 150 bases. Further, the number of base pairs constituting the hairpin structure is preferably approximately 5 to 50.

In the single-stranded oligonucleotide of the present embodiment, it is preferred that a non-complementary base pair is included within the hairpin structure, and the quenching substance is labeled at a nucleotide residue located closer to the 5' side among nucleotide residues constituting the non-complementary base pair. As described later in the examples, a single-stranded oligonucleotide having such a structure tends to have increased efficiency of cleavage by a flap endonuclease.

In the single-stranded oligonucleotide of the present embodiment, the number of non-complementary base pairs may be 1 to 3, preferably 1 or 2, and more preferably 1.

### (Fluorescent Substance)

The fluorescent substance is not particularly limited, and examples thereof include fluorescein, ATTO 425, Alexa 488, ATTO 542, Yakima Y, Redmond R, ATTO 643, Alexa 647, Alexa 680, Alexa 568, FAM, ATTO 633, Cy 5, HiLyte Fluor 647, ATTO 663, and the like.

In the single-stranded oligonucleotide of the present embodiment, it is preferred that the fluorescent substance is labeled at a 5' end nucleotide residue, and an oligonucleotide fragment containing the fluorescent substance is released when cleaved by a flap endonuclease. Here, the released oligonucleotide fragment preferably has a length of 2 or more residues. The length of the released oligonucleotide fragment is preferably 2 or 3 residues, and more preferably 2 residues. As described later in the examples, such a single-stranded oligonucleotide tends to have increased efficiency of cleavage by a flap endonuclease.

### (Quenching Substance)

The quenching substance is not particularly limited as long as it can quench the fluorescence of the fluorescent substance used, and examples thereof include Black Hole Quencher (BHQ) (registered trademark)-1, BHQ (registered trademark)-2, BHQ (registered trademark)-3, Tide Quencher 1 (TQ1), Tide Quencher 2 (TQ2), Tide Quencher 2WS (TQ2WS), Tide Quencher 3 (TQ3), Tide Quencher 3WS (TQ3WS), Tide Quencher 4 (TQ4), Tide Quencher 4WS (TQ4WS), Tide Quencher 5 (TQ5), Tide Quencher 5WS (TQ5WS), Tide Quencher 6WS (TQ6WS), Tide Quencher 7WS (TQ7WS), QSY35, QSY7, QSY9, QSY21, Iowa Black FQ and Iowa Black RQ. The quenching substance is selected that can quench the fluorescence of the fluorescent substance used.

When a fluorescent substance is in spatial proximity to a quenching substance, fluorescence from the fluorescent substance is quenched by the quenching substance. The term "fluorescence is quenched" means as follows. In the absence of the quenching substance, the intensity of fluorescence emitted from the fluorescent substance when the fluorescent substance is irradiated with excitation light is defined as A. Further, in the presence of the quenching substance in spatial proximity to the fluorescent substance, the intensity of fluorescence emitted from the fluorescent substance when the fluorescent substance is irradiated with excitation light is defined as B. Here, the "fluorescence is quenched" means that the value of B/A is 40% or less.

When the fluorescent substance is in spatial proximity to the quenching substance, the distance between the fluorescent substance and the quenching substance is not particularly limited as long as the fluorescence emitted from the fluorescent substance is suppressed by the quenching substance, and is preferably 10 nm or less, more preferably 5 nm or less, and still more preferably 2 nm or less.

### (Oligonucleotide)

The single-stranded oligonucleotide constituting the fluorescent substrate of the present embodiment is not particularly limited as long as the advantageous effects of the present invention are achieved, and may be a natural nucleic acid or a synthetic nucleic acid.

Examples of the natural nucleic acids include genomic DNA, mRNA, rRNA, hnRNA, miRNA and tRNA. The natural nucleic acids may be those recovered from living organisms or may be those recovered from water, organic matter, or the like that has been in contact with living organisms. Methods for recovering natural nucleic acids include known methods such as the phenol/chloroform method.

Examples of the synthetic nucleic acids include synthetic DNA, synthetic RNA, cDNA, bridged nucleic acid (BNA) and locked nucleic acid (LNA).

Methods for synthesizing the nucleic acid are not particularly limited, and examples thereof include known chemical synthesis methods such as the DNA solid-phase synthesis by phosphoramidite method, known nucleic acid amplification methods and reverse transcription reactions. Examples of the nucleic acid amplification reaction include PCR, LAMP, SMAP, NASBA and RCA.

In the fluorescent substrate of the present embodiment, it is preferred that the fluorescent substance is located in proximity to a 5' end nucleotide residue, and the base of the 5' end nucleotide residue is a pyrimidine base. A purine base has a tendency to quench the fluorescence of the fluorescent substance. Therefore, when the base of the 5' end nucleotide residue is a pyrimidine base, quenching of the fluorescence of the fluorescent substance is suppressed, which is preferred.

### [Method of Improving Cleavage Efficiency in Cleavage of Nucleic Acid Triplex Structure with Flap Endonuclease]

In one embodiment, the present invention provides a method for improving cleavage efficiency in cleavage of a nucleic acid triplex structure with a flap endonuclease, the method including a step of bringing the flap endonuclease into contact with the nucleic acid triplex structure, wherein the nucleic acid triplex structure includes a single-stranded oligonucleotide labeled with a fluorescent substance and a quenching substance, and a single-stranded oligonucleotide having a complementary base sequence on the 3' side, the single-stranded oligonucleotide forms a hairpin structure by self-hybridization on the 5' side, and when the single-stranded oligonucleotide having a complementary base sequence on the 3' side hybridizes, a triplex structure is formed on the 5' end portion, and cleaved by the flap endonuclease, whereby the fluorescent substance is released from the quenching substance, the fluorescent substance emitting fluorescence when irradiated with excitation light, and a nucleotide residue labeled with the fluorescent substance and a nucleotide residue labeled with the quenching substance are separated from each other by 4 or more residues.

In the method of the present embodiment, the ICA, fluorescent substance, quenching substance, oligonucleotide, and the like are the same as those described above. The cleavage efficiency in cleavage with a flap endonuclease can be improved by using, as a fluorescent substrate, a single-stranded oligonucleotide in which the nucleotide residue labeled with a fluorescent substance and the nucleotide residue labeled with a quenching substance are separated from each other by 4 or more residues.

In the method of the present embodiment, it is preferred that, in the single-stranded oligonucleotide, the fluorescent substance is labeled at a 5' end nucleotide residue, and an oligonucleotide fragment containing the fluorescent substance is released when cleaved by a flap endonuclease. Here, the released oligonucleotide fragment preferably has a length of 2 or more residues. The length of the released oligonucleotide fragment is preferably 2 or 3 residues, and more preferably 2 residues. As described later in the examples, such a single-stranded oligonucleotide tends to have increased efficiency of cleavage by a flap endonuclease.

In the method of the present embodiment, it is preferred that, in the single-stranded oligonucleotide, the fluorescent substance is located in proximity to a 5' end nucleotide residue, and the base of the 5' end nucleotide residue is a pyrimidine base. As described above, when the base of the 5' end nucleotide residue is a pyrimidine base, quenching of the fluorescence of the fluorescent substance is suppressed, which is preferred.

In the method of the present embodiment, it is preferred that, in the single-stranded oligonucleotide, a non-complementary base pair is included within the hairpin structure, and the quenching substance is labeled at a nucleotide residue located closer to the 5' side among nucleotide residues constituting the non-complementary base pair. As described later in the examples, a single-stranded oligonucleotide having such a structure tends to have increased efficiency of cleavage by a flap endonuclease.

In the method of the present embodiment, ICA is preferably performed in a microspace. Specifically, it is preferred to perform ICA using a device including a well array having a plurality of microwells.

The wells may be used without treatment, or may be pre-treated such as by immobilizing an extraction reagent, a detection reagent such as antibody, or the like on the inner wall of the wells, or covering the well openings with a lipid bilayer depending on the purpose.

The device may have flow channels, and a reaction solution for ICA may be supplied to the wells of the well array through the flow channels.

The well may have a diameter of approximately 3 µm and a depth of approximately 4.5 µm, for example. The wells may be arranged on the substrate member to form a well array arranged in a triangular lattice or a square lattice.

Examples of the material for the substrate member include cycloolefin polymer, cycloolefin copolymer, silicone, polypropylene, polycarbonate, polystyrene, polyethylene, polyvinyl acetate, fluororesin and amorphous fluororesin.

The number of wells in the well array is preferably approximately 100,000 to 6,000,000 per device. The volume per well is preferably 1 fL to 6 pL.

### [Examples]

The present invention will be described below by way of examples, but the present invention should not be limited to the examples below.

### [Experimental Example A1]

A reaction solution containing a plurality of types of target nucleic acids, allele probes, invader oligos and FRET cassettes were prepared, and ICA was performed to detect red fluorescence.

The target nucleic acids, allele probes, invader oligos and FRET cassettes shown in Table 1 below were prepared.

**[Table 1]**

| SEQ ID NO | Name | Base sequence (5' - 3') |
|---|---|---|
| 1 | FRET cassette 1 | XYTTCTZAGCCGGTTTTCCGGCTGAGACCTCGGCGCG X: Alexa Fluor 488, Y: Amino-Modifier C6, Z: BHQ-1-dT |
| 2 | FRET cassette 2 | XYTTCTZAGCCGGTTTTCCGGCTGAGACGGCCTCGCG X: ATTO 542, Y: Amino-Modifier C6, Z: BHQ-1-dT |
| 3 | FRET cassette 3 | XYTTCTZAGCCGGTTTTCCGGCTGAGACTCCGCGTCCGT X: Alexa Fluor 568, Y: Amino-Modifier C6, Z: BHQ-2-dT |
| 4 | FRET cassette 4-1 | XYTTCTZAGCCGGTTTTCCGGCTGAGACGATGCGAGCTC A X: ATTO 643, Y: Amino-Modifier C6, Z: BHQ-2-dT |
| 5 | FRET cassette 4-2 | XYTTCTZAGCCGGTTTTCCGGCTGAGACGATGCGAGCTC A X: ATTO 643, Y: Amino-Modifier C6, Z: BHQ-1-dT |
| 6 | FRET cassette 4-3 | XYTTCTZAGCCGGTTTTCCGGCTGAGACGATGCGAGCTC A X: ATTO 643, Y: Amino-Modifier C6, Z: BHQ-3-dT |
| 7 | Target nucleic acid 1 | CTTTGTGTTCCCGGACATAGTCCAGGAGGCAGCCGAAggg catgagctgcGTGATGAGCTGCACGGTGGAGGTGAGGCAGAT GCCCAGCAGGCGGCACACG |
| 8 | Target nucleic acid 2 | CTTTGTGTTCCCGGACATAGTCCAGGAGGCAGCCGAAggg catgagctgcATGATGAGCTGCACGGTGGAGGTGAGGCAGAT GCCCAGCAGGCGGCACACG |
| 9 | Target nucleic acid 3 | GCCAGGAACGTACTGGTGAAAACACCGCAGCATGTCaaga tcacagattttgggcTGGCCAAACTGCTGGGTGCGGAAGAGAAA GAATACCATGCAGAAGG |
| 10 | Target nucleic acid 4 | GCCAGGAACGTACTGGTGAAAACACCGCAGCATGTCaaga tcacagattttgggcGGGCCAAACTGCTGGGTGCGGAAGAGAAA GAATACCATGCAGAAGG |
| 11 | Allele probe 1 | CGCGCCGAGGCgcagctcatgccc |
| 12 | Allele probe 2 | ACGGACGCGGAGTgcagctcatgccc |
| 13 | Invader oligo 1 | TCTGCCTCACCTCCACCGTGCARCTCATCAA |
| 14 | Allele probe 3 | CGCGAGGCCGAgcccaaaatctgtgatctt |
| 15 | Allele probe 4 | TGAGCTCGCATCGCgcccaaaatctgtgatctt |
| 16 | Invader oligo 2 | CGCACCCAGCAGTTTGGCCA |

Target nucleic acid 1 and target nucleic acid 2 were target nucleic acids having the same base sequence except for one underlined base. Target nucleic acid 3 and target nucleic acid 4 were target nucleic acids having the same base sequence except for one underlined base.

Target nucleic acid 1 was detected by allele probe 1 capable of hybridizing to target nucleic acid 1, invader oligo 1 and FRET cassette 1. The lowercase bases in target nucleic acid 1 and allele probe 1 were base pairs capable of hybridizing to each other.

Target nucleic acid 2 was detected by allele probe 2 capable of hybridizing to target nucleic acid 2, invader oligo 1 and FRET cassette 3. The lowercase bases in target nucleic acid 2 and allele probe 2 were base pairs capable of hybridizing to each other.

Target nucleic acid 3 was detected by allele probe 3 capable of hybridizing to target nucleic acid 3, invader oligo 2 and FRET cassette 2. The lowercase bases in target nucleic acid 3 and allele probe 3 were base pairs capable of hybridizing to each other.

Target nucleic acid 4 was detected by allele probe 4 capable of hybridizing to target nucleic acid 4, invader oligo 2 and FRET cassette 4-1, 4-2 or 4-3. The lowercase bases in target nucleic acid 4 and allele probe 4 were base pairs capable of hybridizing to each other.

The fluorescent substance and the quenching substance in each FRET cassette were as follows:
FRET cassette 1: Alexa Fluor 488, BHQ-1
FRET cassette 2: ATTO 542, BHQ-1
FRET cassette 3: Alexa Fluor 568, BHQ-2
FRET cassette 4-1: ATTO 643, BHQ-2
FRET cassette 4-2: ATTO 643, BHQ-1
FRET cassette 4-3: ATTO 643, BHQ-3

Reagents were mixed as shown in Table 2 below to prepare reaction solutions 1 to 3.

**[Table 2]**

| Reagent | Final concentration |
|---|---|
| Allele probe 1 | 2 µM |
| Allele probe 2 | 1 µM |
| Invader oligo 1 | 1 µM |
| Allele probe 3 | 2 µM |
| Allele probe 4 | 2 µM |
| Invader oligo 2 | 1 µM |
| FRET cassette 1 | 4 µM |
| FRET cassette 2 | 4 µM |
| FRET cassette 3 | 4 µM |
| FRET cassette 4-1 or 4-2 or 4-3 | 4 µM |
| FEN-1 | 0.4 mg/mL |
| MgCl₂ | 25 mM |
| Tris pH8.5 | 50 mM |
| Tween 20 | 0.05 % |
| Target nucleic acid mix | 15 fM each |
| DW | up to 10 µL |

The reaction solutions 1 to 3 each contained one selected from FRET cassette 1, FRET cassette 2, FRET cassette 3, FRET cassette 4-1, FRET cassette 4-2 and FRET cassette 4-3.

The reaction solution 1 contained FRET cassette 4-1, the reaction solution 2 contained FRET cassette 4-2, and the reaction solution 3 contained FRET cassette 4-3.

### (Measuring Device)

A substrate member having a large number of microwells was formed of COP (cycloolefin polymer), and a lid member made of COP was attached to the substrate member to produce a measuring device. The total volume of wells per cm² was 0.93 µL. The total number of wells used in measurement was 1,000,000.

### <Supply of Reaction Mixture Solution>

8 µL of the reaction solution 1 was supplied into the device, and introduced into the respective wells. Then, 200 µL. of FC-40 (Sigma) was supplied as a sealing solution to seal the respective wells. Thus, one or less target nucleic acid per well was sealed in each well. That is, each well contained target nucleic acid or did not contain target nucleic acid.

### <Nucleic Acid Detection Reaction>

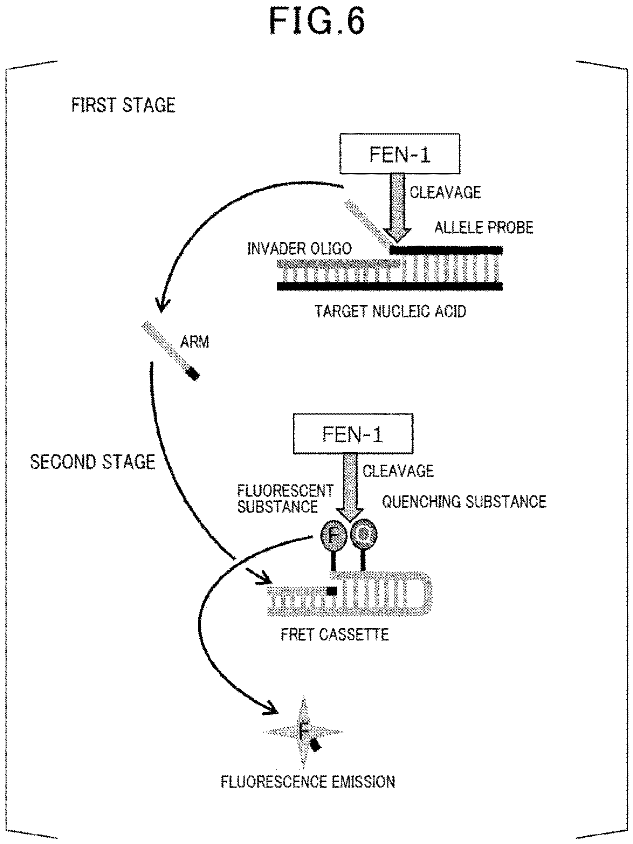

The above device after the supply of reaction mixture solution was set on a hot plate, and allowed to react at 66°C for 25 minutes. As a result, recognition of the target nucleic acid by the allele probe and the invader oligo, cleavage of the allele probe by FEN-1, binding of the released allele probe fragment to the FRET cassette, and cleavage of the FRET cassette by FEN-1 proceeded, and a fluorescence signal was generated. Fig. 6 shows a schematic diagram of this reaction process.

### (Fluorescence Intensity Measurement)

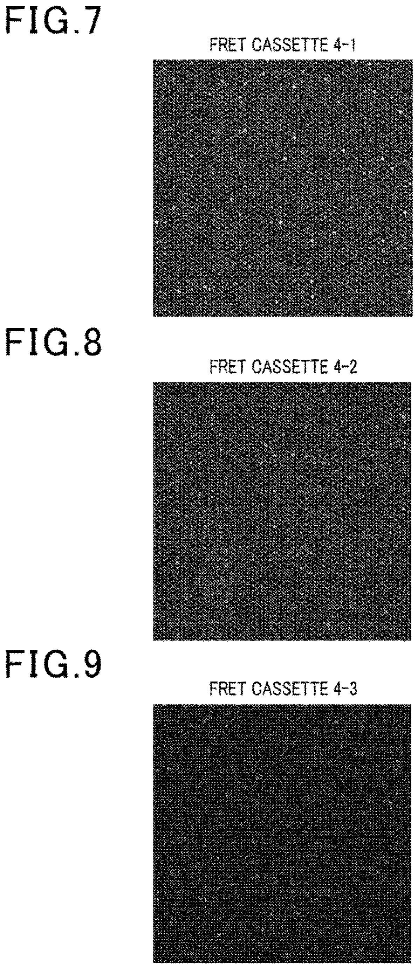

After heating the device 1 containing the reaction solution 1 at 66°C for 25 minutes, a fluorescence signal image of the wells in the device was acquired using a fluorescence microscope (BZ-700, KEYENCE Corporation) with a 4x objective lens and a fluorescence filter (excitation: 607 mn/70 nm, dichroic mirror: 665 nm, fluorescence: 700 nm/75 nm), and an exposure time set to 2 seconds. Fig. 7 shows the results. Further, Figs. 8 and 9 show fluorescence signal images obtained in the same manner as above except that the reaction solutions 2 and 3 were used, respectively.

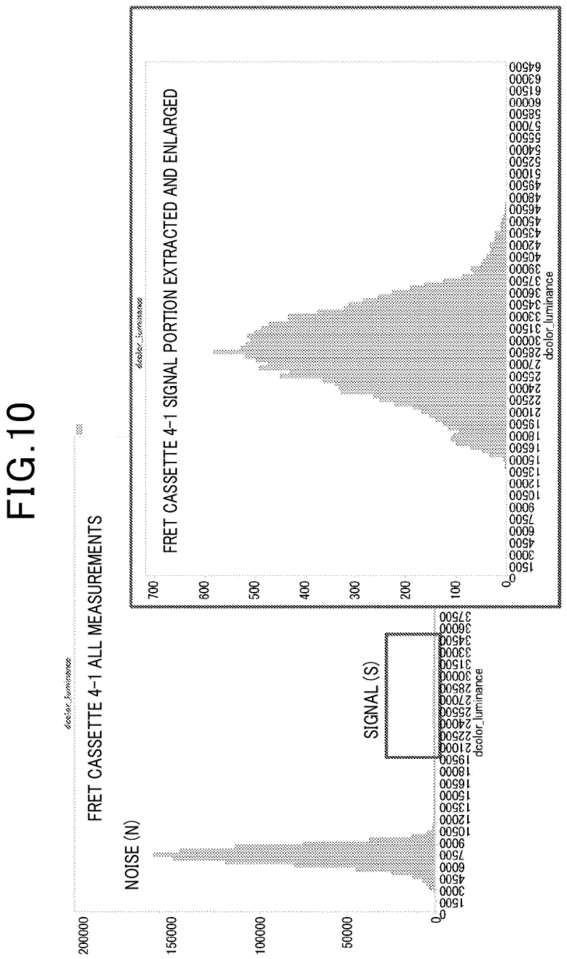

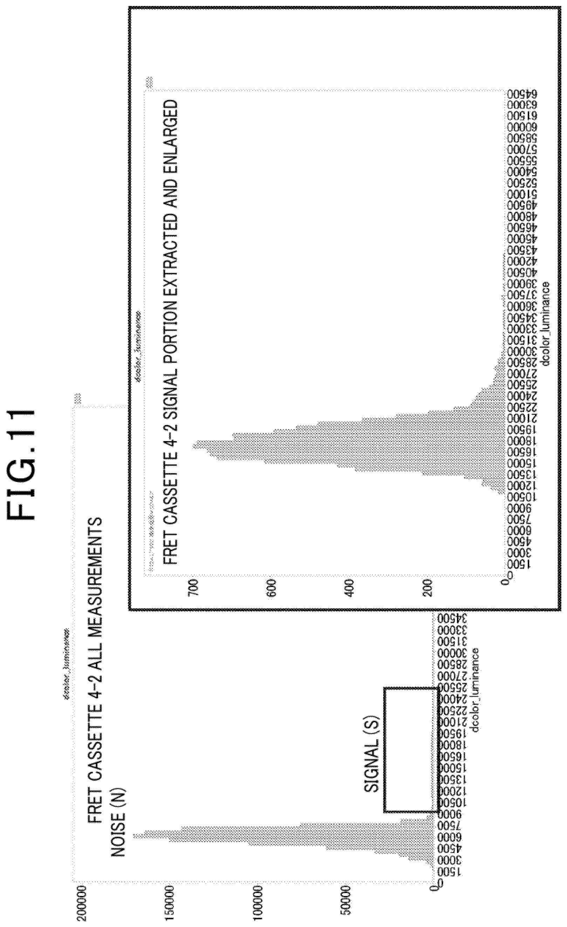

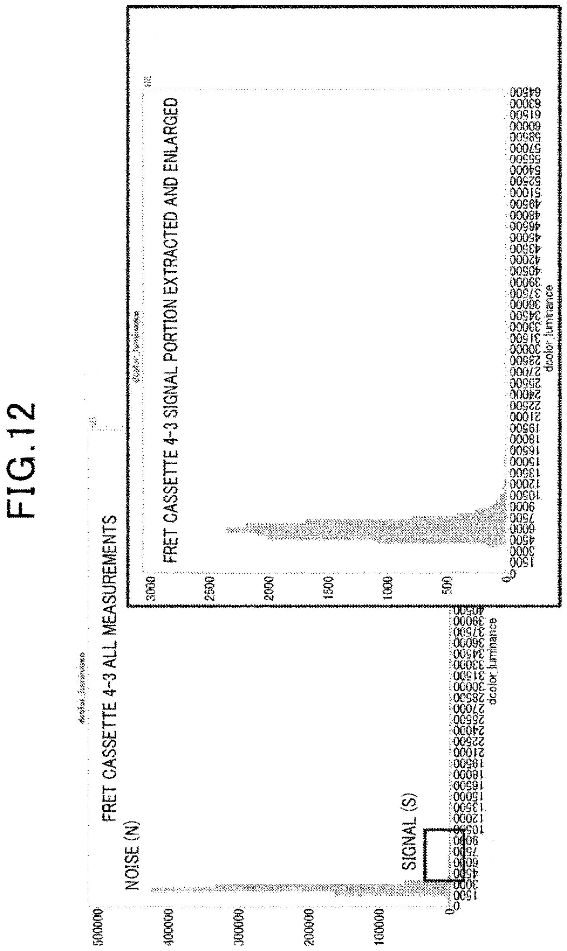

Figs. 10 to 12 show the measurement results of the fluorescence intensity of each reaction solution. Fig. 10 shows the measurement results of the fluorescence intensity of the reaction solution 1, Fig. 11 shows the measurement results of the fluorescence intensity of the reaction solution 2, and Fig. 12 shows the measurement results of the fluorescence intensity of the reaction solution 3.

In Fig. 10, a fluorescence intensity of 3,000 to 12,000 was determined to be noise (N), and a fluorescence intensity of 15,000 to 45,000 was determined to be fluorescence signal (S). In Fig. 11, a fluorescence intensity of 1,500 to 9,000 was determined to be noise (N), and a fluorescence intensity of 12,000 to 42,000 was determined to be fluorescence signal (S). In Fig. 12, a fluorescence intensity of 1,000 to 3,000 was determined to be noise (N), and a fluorescence intensity of 30,000 to 12,000 was determined to be fluorescence signal (S).

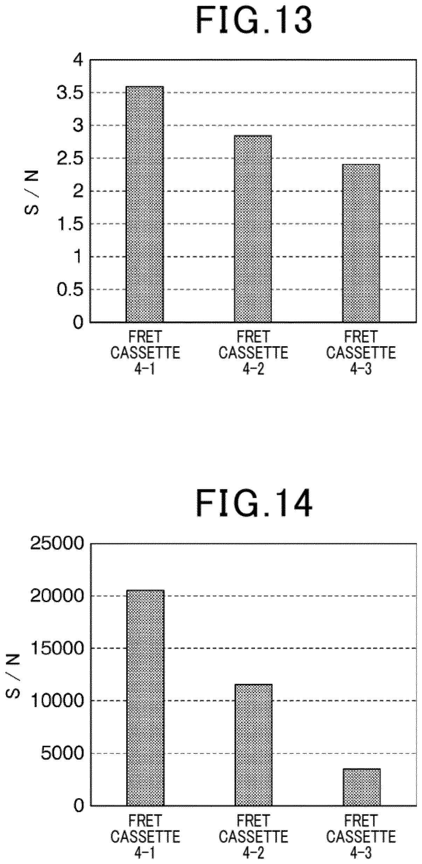

According to the above determination criteria, the average and standard deviation were calculated for the noise (N) and fluorescence signal (S). Further, S/N value (that is, the value obtained by dividing the average of S by the average of N) and S-N value (that is, difference between the average of S and the average of N) were calculated. The results are shown in Table 3. Fig. 13 shows the graph of S/N value, and Fig. 14 shows the graph of S-N value.

**[Table 3]**

| | Trial | N average | N standard deviation | S average | S standard deviation | S/N | S-N |
|---|---|---|---|---|---|---|---|
| FRET cassette 4-1 | N1 | 7,715 | 3,052 | 28,149 | 5,356 | 3.65 | 20,434 |
| | N2 | 7,925 | 3,102 | 29,007 | 5,539 | 3.66 | 21,082 |
| | N3 | 8,229 | 2,928 | 28,319 | 5,052 | 3.44 | 20,090 |
| FRET cassette 4-2 | N1 | 6,409 | 1,804 | 17,407 | 2,421 | 2.72 | 10,998 |
| | N2 | 5,936 | 1,907 | 17,569 | 3,312 | 2.96 | 11,633 |
| | N3 | 6,715 | 1,811 | 18,778 | 3,120 | 2.80 | 12,063 |
| FRET cassette 4-3 | N1 | 2,590 | 647 | 5,889 | 1,135 | 2.27 | 3,299 |
| | N2 | 2,174 | 624 | 5,683 | 1,097 | 2.61 | 3,509 |
| | N3 | 2,568 | 631 | 6,115 | 1,159 | 2.38 | 3,547 |

Among FRET cassettes 4-1, 4-2 and 4-3, both the S/N value and S-N value were highest when 4-1 was used.

For ATTO 643, BHQ-3 is recommended as a quenching substance. However, unexpectedly, BHQ-2 had higher S/N value and S-N value than BHQ-3 as described above. Therefore, from the viewpoint of more accurate discrimination between noise and signal, it was found that BHQ-2 is preferably used as the quenching substance when ATTO 643 is used as the fluorescent substance.

### [Experimental Example A2]

### (Fluorescent Substrate Study)

ICA was performed using a fluorescent substrate in which the fluorescent substance was bound to the base and a fluorescent substrate in which the fluorescent substance was bound to a portion other than the base, and reactivity was compared.

ICA was performed using the nucleic acid fragments shown in Tables 4 and 5 below. The target nucleic acid (SEQ ID NO: 17) in Table 4 was the nucleic acid to be detected. In this example, ICA detected the presence of g (guanine) indicated by a lowercase letter in the target nucleic acid. The flap probe (SEQ ID NO: 18) and the invasion probe (SEQ ID NO: 19) each had a base sequence complementary to the target nucleic acid (SEQ ID NO: 17).

When the flap probe (SEQ ID NO: 18) and the invasion probe (SEQ ID NO: 19) each hybridize to the target nucleic acid (SEQ ID NO: 17), a triplex structure is formed. Here, the lowercase portion "5'-cgcgccgaggc-3"' (SEQ ID NO: 20) in the flap probe (SEQ ID NO: 18) does not form a base pair, and forms a flap site.

The flap endonuclease recognizes the above triplex structure and cleaves the flap probe (SEQ ID NO: 18), whereby the nucleic acid fragment (SEQ ID NO: 20) is excised.

**[Table 4]**

| Name | SEQ ID NO | Base sequence (5' → 3') |
|---|---|---|
| Target nucleic acid | 17 | CCGAAGGGCATGAGCTGCgTGATGAGCTGCACGGTGGA GGTGAGGCAGATGCCCAGCAGGCGGCACACGTGGGGGT TGTCCACGCTGGCCATCACGTAGGCTTCC |
| Flap probe | 18 | cgcgccgaggcGCAGCTCATGCCC |
| Invasion probe | 19 | CCACCGTGCAGCTCATCAA |
| Nucleic acid fragment | 20 | cgcgccgaggc |

**[Table 5]**

| Name | SEQ ID NO | Base sequence (5' → 3') |
|---|---|---|
| Fluorescent substrate 1 | 21 | F-TCT-T(Q)-AGCCGGTTTTCCGGCTGAGACCTCGGCGCG |
| Fluorescent substrate 2 | 22 | F-TCT-T(Q)-AGCCGGTTTTCCGGCTGAGACCTCGGCGCG |

The fluorescent substrate corresponds to the above-mentioned FRET cassette. Fluorescent substrate 1 (SEQ ID NO: 21) shown in Table 5 was a fluorescent substrate in which the fluorescent substance was bound to the base, and specifically had a structure shown in the following formula (A5). In fluorescent substrate 1, F indicates fluorescein, and Q indicates BHQ (registered trademark)-1.

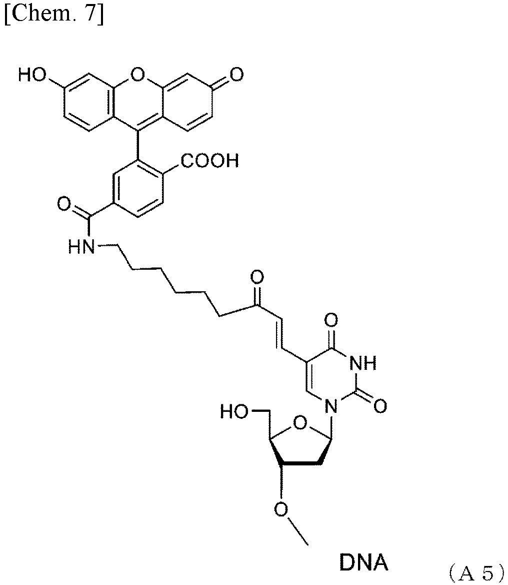

Fluorescent substrate 2 (SEQ ID NO: 22) shown in Table 5 was a fluorescent substrate in which the fluorescent substance was bound to a portion other than the base, and specifically had a structure shown in the following formula (A6). In the fluorescent substrate 2, F indicates fluorescein, and Q indicates BHQ (registered trademark)-1.

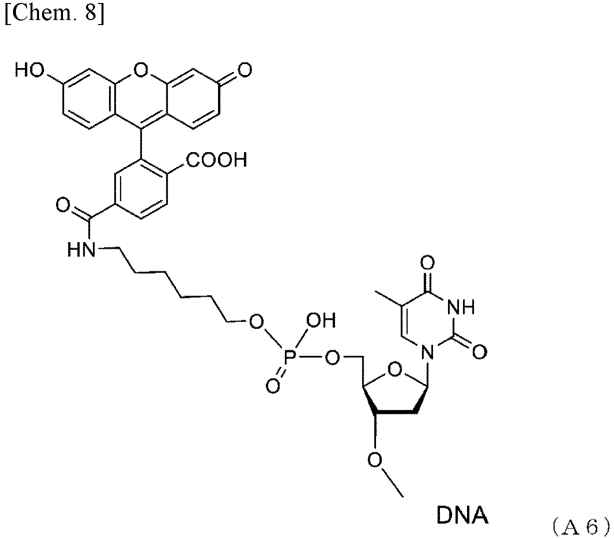

When the excised nucleic acid fragment (SEQ ID NO: 20) hybridizes to fluorescent substrate 1 or fluorescent substrate 2, the T (thymine) residue shown in bold in fluorescent substrate 1 or fluorescent substrate 2 in Table 5 forms a triplex structure that serves as a substrate for flap endonuclease, which is then cleaved. As a result, the fluorescent substance is separated from the quenching substance, and a fluorescence signal is detected upon irradiation with excitation light.

First, a reaction solution was prepared with the composition shown in Table 6 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 6]**

| Reagent | Final concentration |
|---|---|
| Target nucleic acid | 1 µM |
| Flap probe | 1 µM |
| Invasion probe | 1 µM |
| Fluorescent substrate 1 or fluorescent substrate 2 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

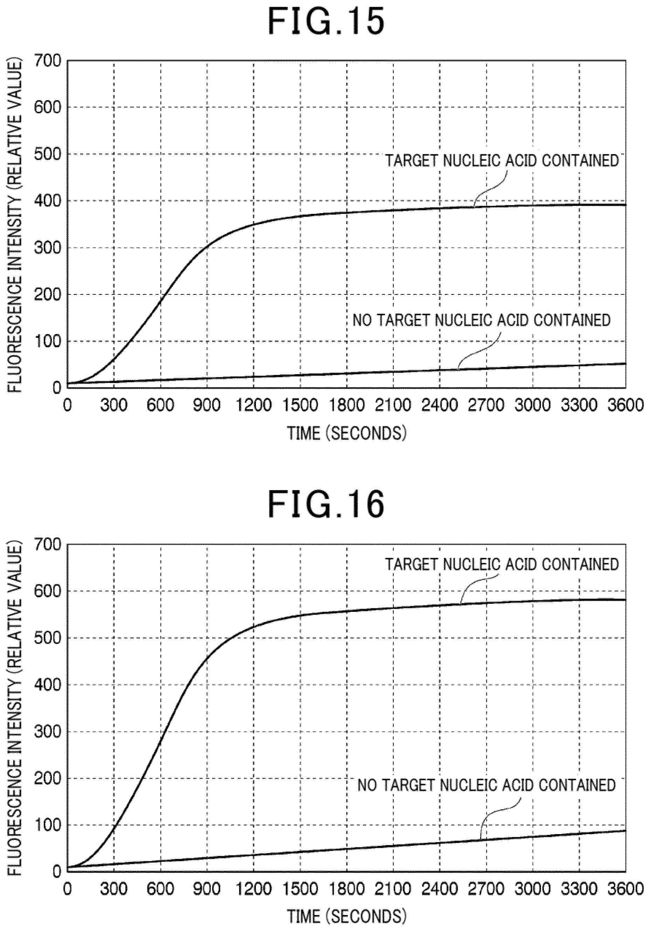

Fig. 15 is a graph showing the results of measuring the change in fluorescence intensity when fluorescent substrate 1 was used. Further, Fig. 16 is a graph showing the results of measuring the change in fluorescence intensity when fluorescent substrate 2 was used. In Figs. 15 and 16, the horizontal axis represents the time (seconds) elapsed from the start of reaction, and the vertical axis represents the fluorescence intensity (relative value).

The results show that the use of fluorescent substrate 2, in which the fluorescent substance is bound to a portion other than the base, has higher reactivity in ICA.

### [Experimental Example B1]

### (Fluorescent Substrate Study 1)

ICA was performed using a fluorescent substrate in which the fluorescent substance was bound to the base and a fluorescent substrate in which the fluorescent substance was bound to a portion other than the base, and reactivity was compared.

ICA was performed using the nucleic acid fragments shown in Tables 7 and 8 below. The target nucleic acid (SEQ ID NO: 23) in Table 7 was the nucleic acid to be detected. In this example, ICA detected the presence of g (guanine) indicated by a lowercase letter in the target nucleic acid. The flap probe (SEQ ID NO: 24) and the invasion probe (SEQ ID NO: 25) each had a base sequence complementary to the target nucleic acid (SEQ ID NO: 23).

When the flap probe (SEQ ID NO: 24) and the invasion probe (SEQ ID NO: 25) each hybridize to the target nucleic acid (SEQ ID NO: 23), a triplex structure is formed. Here, the lowercase portion "5'-cgcgccgaggc-3"' (SEQ ID NO: 26) in the flap probe (SEQ ID NO: 24) does not form a base pair, and forms a flap site.

The flap endonuclease recognizes the above triplex structure and cleaves the flap probe (SEQ ID NO: 24), whereby the nucleic acid fragment (SEQ ID NO: 26) is excised.

**[Table 7]**

| Name | SEQ ID NO | Base sequence (5' → 3') |
|---|---|---|
| Target nucleic acid | 23 | CCGAAGGGCATGAGCTGCgTGATGAGCTGCACGGTGGAGGTG AGGCAGATGCCCAGCAGGCGGCACACGTGGGGGTTGTCCACG CTGGCCATCACGTAGGCTTCC |
| Flap probe | 24 | cgcgccgaggcGCAGCTCATGCCC |
| Invasion probe | 25 | CCACCGTGCAGCTCATCAA |
| Nucleic acid fragment | 26 | cgcgccgaggc |

**[Table 8]**

| Name | SEQ ID NO | Base sequence (5' → 3') |
|---|---|---|
| Fluorescent substrate B1 | 27 | F-**T**CT-T(Q)-AGCCGGTTTTCCGGCTGAGACCTCGGCGCG |
| Fluorescent substrate B2 | 28 | F-**T**CT-T(Q)-AGCCGGTTTTCCGGCTGAGACCTCGGCGCG |

Fluorescent substrate B1 (SEQ ID NO: 27) shown in Table 8 was a fluorescent substrate in which the fluorescent substance was bound to the base, and specifically had a structure shown in the following formula (B5). In fluorescent substrate B1, F indicates fluorescein, and Q indicates BHQ (registered trademark)-1.

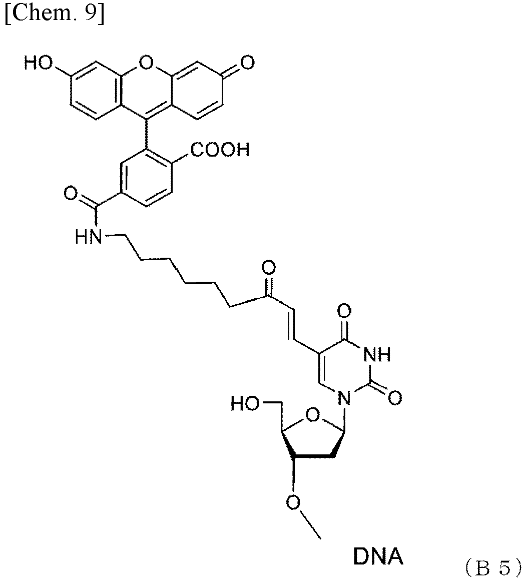

Fluorescent substrate B2 (SEQ ID NO: 28) shown in Table 8 was a fluorescent substrate in which the fluorescent substance was bound to a portion other than the base, and specifically had a structure shown in the following formula (B6). In fluorescent substrate B2, F indicates fluorescein, and Q indicates BHQ (registered trademark)-1.

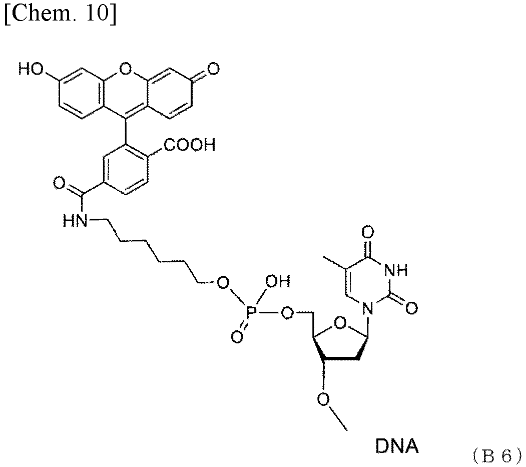

When the excised nucleic acid fragment (SEQ ID NO: 4) hybridizes to fluorescent substrate B1 or fluorescent substrate B2, the T (thymine) residue shown in bold in fluorescent substrate B1 or fluorescent substrate B2 in Table 8 forms a triplex structure that serves as a substrate for flap endonuclease, which is then cleaved. As a result, the fluorescent substance is separated from the quenching substance, and a fluorescence signal is detected upon irradiation with excitation light.

First, a reaction solution was prepared with the composition shown in Table 9 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 9]**

| Reagent | Final concentration |
|---|---|
| Target nucleic acid | 1 µM |
| Flap probe | 1 µM |
| Invasion probe | 1 µM |
| Fluorescent substrate B1 or fluorescent substrate B2 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

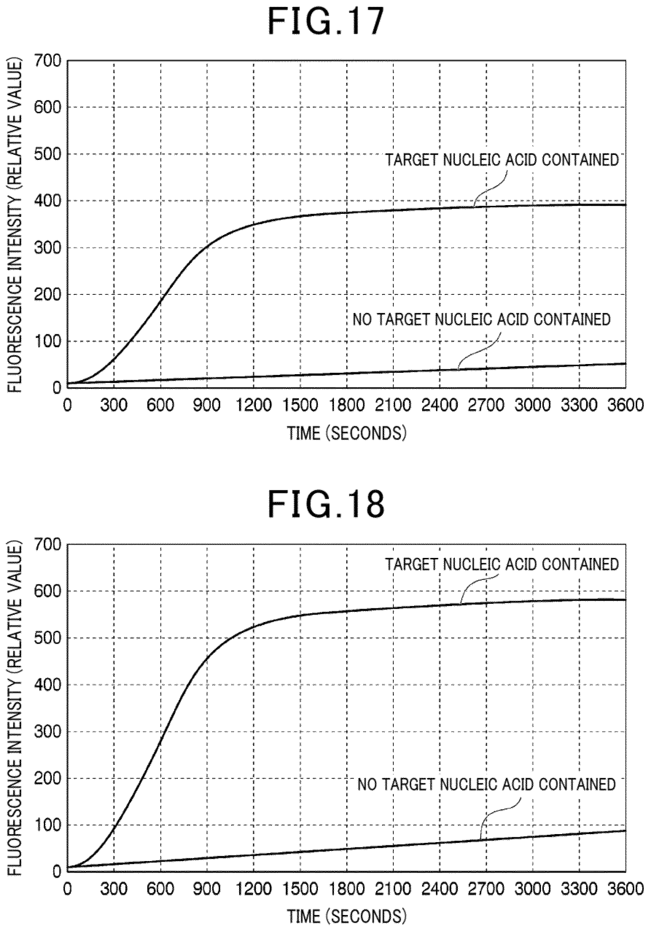

Fig. 17 is a graph showing the results of measuring the change in fluorescence intensity when fluorescent substrate B1 was used. Further, Fig. 18 is a graph showing the results of measuring the change in fluorescence intensity when fluorescent substrate B2 was used. In Figs. 17 and 18, the horizontal axis represents the time (seconds) elapsed from the start of reaction, and the vertical axis represents the fluorescence intensity (relative value).

The results show that the use of fluorescent substrate B2, in which the fluorescent substance is bound to a portion other than the base, has higher reactivity in ICA.

### [Experimental Example B2]

### (Fluorescent Substrate Study 2)

ICA was performed using a fluorescent substrate in which the quenching substance was incorporated into the sugar-phosphate backbone and a fluorescent substrate in which the quenching substance was bound to a portion other than the base, and reactivity was compared.

The target nucleic acid (SEQ ID NO: 23), the flap probe (SEQ ID NO: 24) and the invasion probe (SEQ ID NO: 25) were the same as those used in Experimental Example B1, and ICA was performed using fluorescent substrate B3 and fluorescent substrate B4 shown in Table 10 below.

**[Table 10]**

| Name | SEQ ID NO | Base sequence (5' → 3') |
|---|---|---|
| Fluorescent substrate B3 | 29 | F-**T**CT-Q-AGCCGGTTTTCCGGCTGAGACCTCGGCGCG |
| Fluorescent substrate B4 | 30 | F-**T**CT-T(Q)-AGCCGGTTTTCCGGCTGAGACCTCGGCGCG |

Fluorescent substrate B3 (SEQ ID NO: 29) shown in Table 10 was a fluorescent substrate in which the quenching substance was incorporated into the sugar-phosphate backbone, and the quenching substance had a structure shown in the following formula (B7). In fluorescent substrate B3, F indicates fluorescein, and Q indicates BHQ (registered trademark)-1.

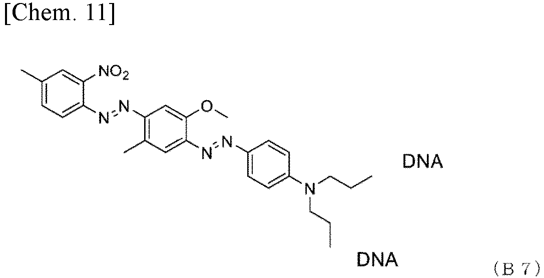

Fluorescent substrate B4 (SEQ ID NO: 30) shown in Table 10 was a fluorescent substrate in which the quenching substance was bound to the base, and the quenching substance had a structure shown in the following formula (B8). In fluorescent substrate B4, F indicates fluorescein, and Q indicates BHQ (registered trademark)-1.

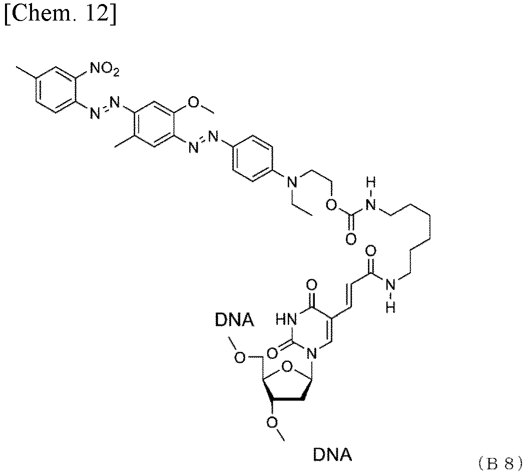

When the nucleic acid fragment (SEQ ID NO: 26) excised from the flap probe (SEQ ID NO: 24) hybridizes to fluorescent substrate B3 or fluorescent substrate B4, the T (thymine) residue shown in bold in fluorescent substrate B3 or fluorescent substrate B4 forms a triplex structure that serves as a substrate for the flap endonuclease, which is then cleaved. As a result, the fluorescent substance is separated from the quenching substance, and a fluorescence signal is detected upon irradiation with excitation light.

First, a reaction solution was prepared with the composition shown in Table 11 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 11]**

| Reagent | Final concentration |
|---|---|
| Target nucleic acid | 1 µM |
| Flap probe | 1 µM |
| Invasion probe | 1 µM |
| Fluorescent substrate B3 or fluorescent substrate B4 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

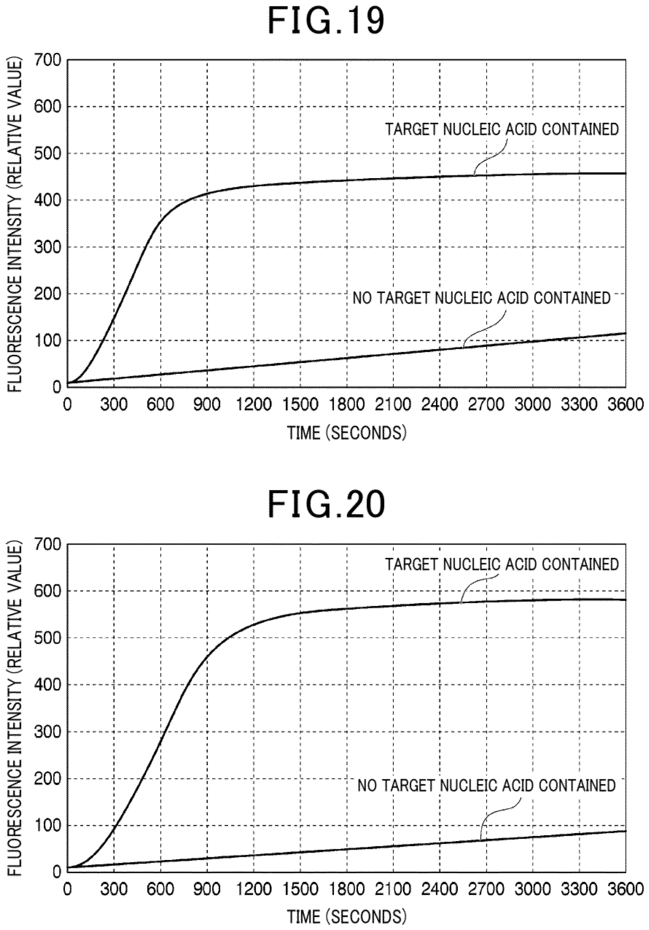

Fig. 19 is a graph showing the results of measuring the change in fluorescence intensity when fluorescent substrate B3 was used. Further, Fig. 20 is a graph showing the results of measuring the change in fluorescence intensity when fluorescent substrate B4 was used. In Figs. 19 and 20, the horizontal axis represents the time (seconds) elapsed from the start of reaction, and the vertical axis represents the fluorescence intensity (relative value).

The results show that the use of fluorescent substrate B4, in which the quenching substance is bound to the base, has higher reactivity in ICA.

### [Experimental Example B3]

### (Fluorescent Substrate Study 3)

ICA was performed using a fluorescent substrate in which a quenching substance is located on the 5' side of a fluorescent substance and a fluorescent substrate in which a quenching substance is located on the 3' side of a fluorescent substance, and reactivity was compared.

The target nucleic acid (SEQ ID NO: 23), the flap probe (SEQ ID NO: 24) and the invasion probe (SEQ ID NO: 25) were the same as those used in Experimental Example B1, and ICA was performed using fluorescent substrate B5 and fluorescent substrate B6 shown in Table 12 below.

**[Table 12]**

| Name | SEQ ID NO | Base sequence (5' → 3') |
|---|---|---|
| Fluorescent substrate B5 | 31 | T(Q)-**T**CT-T(F)-AGCCGGTTTTCCGGCTGAGACCTCGGCGCG |
| Fluorescent substrate B6 | 32 | F-**T**CT-T(Q)-AGCCGGTTTTCCGGCTGAGACCTCGGCGCG |

Fluorescent substrate B5 (SEQ ID NO: 31) shown in Table 12 was a fluorescent substrate in which a quenching substance was located on the 5' side of a fluorescent substance, and specifically had a structure shown in the following formula (B9). In fluorescent substrate B5, F indicates fluorescein, and Q indicates BHQ (registered trademark)-1. In the following formula (B9), Q modifies the base of the thymine residue at the 5' end, and F modifies the base of the thymine residue in the chain.

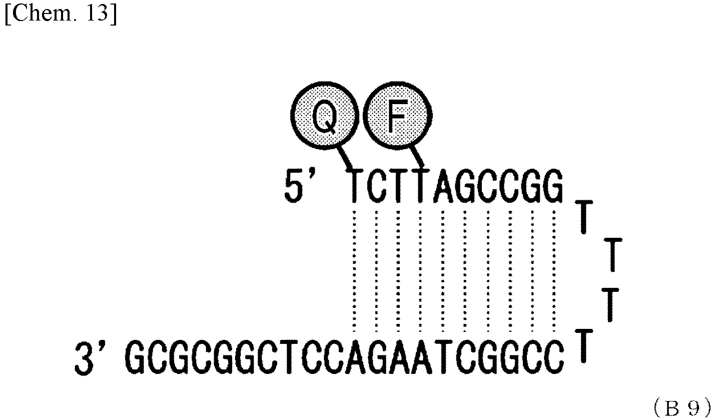

Fluorescent substrate B6 (SEQ ID NO: 32) shown in Table 12 was a fluorescent substrate in which a quenching substance was located on the 3' side of a fluorescent substance, and specifically had a structure shown in the following formula (B10). In fluorescent substrate B6, F indicates fluorescein, and Q indicates BHQ (registered trademark)-1. In the following formula (B10), F modifies the base of the thymine residue at the 5' end, and Q modifies the base of the thymine residue in the chain.

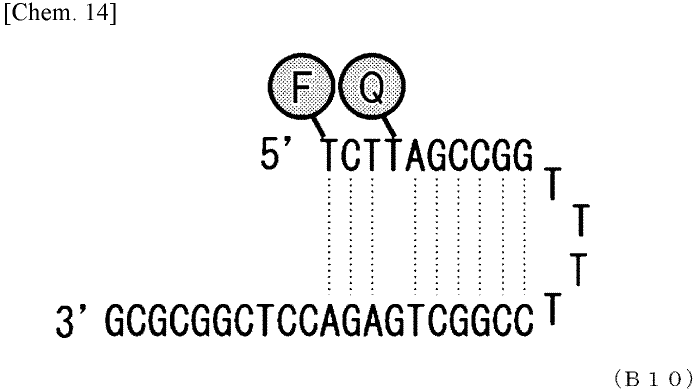

When the nucleic acid fragment (SEQ ID NO: 26) excised from the flap probe (SEQ ID NO: 24) hybridizes to fluorescent substrate B5 or fluorescent substrate B6, the T (thymine) residue shown in bold in fluorescent substrate B5 or fluorescent substrate B6 forms a triplex structure that serves as a substrate for flap endonuclease, which is then cleaved. As a result, the fluorescent substance is separated from the quenching substance, and a fluorescence signal is detected upon irradiation with excitation light.

First, a reaction solution was prepared with the composition shown in Table 13 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 13]**

| Reagent | Final concentration |
|---|---|
| Target nucleic acid | 1 µM |
| Flap probe | 1 µM |
| Invasion probe | 1 µM |
| Fluorescent substrate B5 or fluorescent substrate B6 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

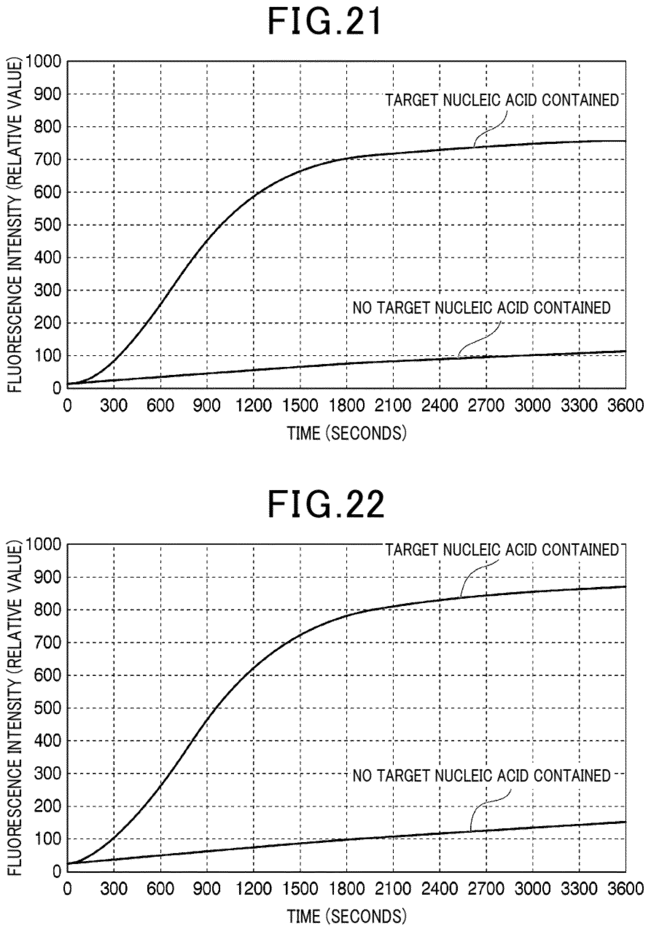

Fig. 21 is a graph showing the results of measuring the change in fluorescence intensity when fluorescent substrate B5 was used. Further, Fig. 22 is a graph showing the results of measuring the change in fluorescence intensity when fluorescent substrate B6 was used. In Figs. 21 and 22, the horizontal axis represents the time (seconds) elapsed from the start of reaction, and the vertical axis represents the fluorescence intensity (relative value).

The results show that the use of fluorescent substrate B6, in which the quenching substance is located on the 3' side of the fluorescent substance, has higher reactivity in ICA.

### [Experimental Example B4]

### (Fluorescent Substrate Study 4)

ICA reactions were performed using a fluorescent substrate in which a fluorescent substance is present in one strand of the double-stranded oligonucleotide portion of the hairpin structure and a quenching substance is present in the other strand, and a fluorescent substrate in which both a fluorescent substance and a quenching substance are present in one strand of the double-stranded oligonucleotide portion of the hairpin structure, and reactivity was compared.

The target nucleic acid (SEQ ID NO: 23), the flap probe (SEQ ID NO: 24) and the invasion probe (SEQ ID NO: 25) were the same as those used in Experimental Example B1, and ICA was performed using the fluorescent substrates shown in Table 14 below.

**[Table 14]**

| Name | SEQ ID NO | Base sequence (5' → 3') |
|---|---|---|
| Fluorescent substrate B7 | 33 | F-**T**CTGAGCCGGTTTTCCGGCT-T(Q)-AGACCTCGGCGCG |
| Fluorescent substrate B8 | 34 | F-**T**CT-T(Q)-AGCCGGTTTTCCGGCTGAGACCTCGGCGCG |

Fluorescent substrate B7 (SEQ ID NO: 33) shown in Table 14 was a fluorescent substrate in which a fluorescent substance was present in one strand of the double-stranded oligonucleotide portion of the hairpin structure and a quenching substance was present in the other strand, and specifically had a structure shown in the following formula (B11). In fluorescent substrate B7, F indicates fluorescein, and Q indicates BHQ (registered trademark)-1. In the following formula (B11), F modifies the base of the thymine residue at the 5' end, and Q modifies the base of the thymine residue in the chain.

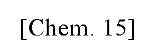

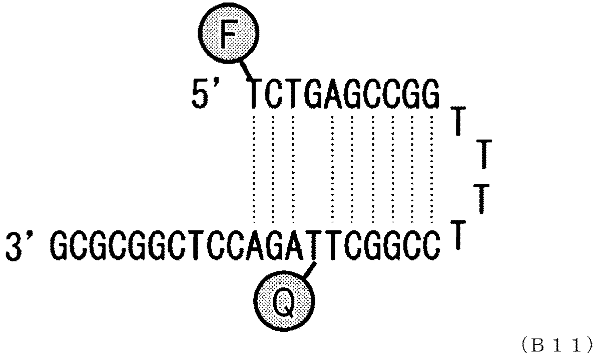

Fluorescent substrate B8 (SEQ ID NO: 34) shown in Table 14 was a fluorescent substrate in which both a fluorescent substance and a quenching substance were present in one strand of the double-stranded oligonucleotide portion of the hairpin structure, and specifically had a structure shown in the following formula (B12). In fluorescent substrate B8, F indicates fluorescein, and Q indicates BHQ (registered trademark)-1. In the following formula (B12), F modifies the base of the thymine residue at the 5' end, and Q modifies the base of the thymine residue in the chain.

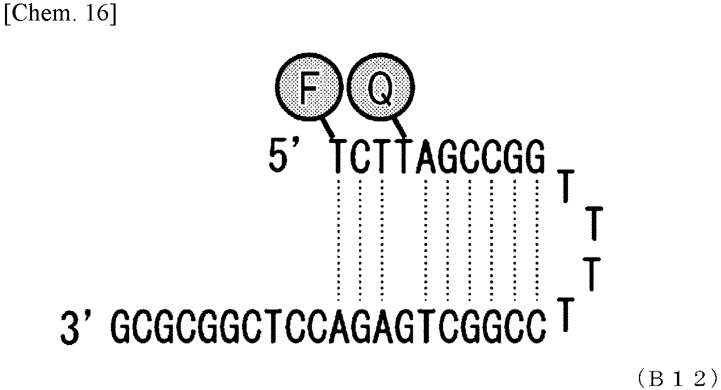

When the nucleic acid fragment (SEQ ID NO: 26) excised from the flap probe (SEQ ID NO: 24) hybridizes to fluorescent substrate 7 or fluorescent substrate B8, the T (thymine) residue shown in bold in fluorescent substrate B7 or fluorescent substrate B8 forms a triplex structure that serves as a substrate for flap endonuclease, which is then cleaved. As a result, the fluorescent substance is separated from the quenching substance, and a fluorescence signal is detected upon irradiation with excitation light.

First, a reaction solution was prepared with the composition shown in Table 15 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 15]**

| Reagent | Final concentration |
|---|---|
| Target nucleic acid | 1 µM |
| Flap probe | 1 µM |
| Invasion probe | 1 µM |
| Fluorescent substrate B7 or fluorescent substrate B8 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

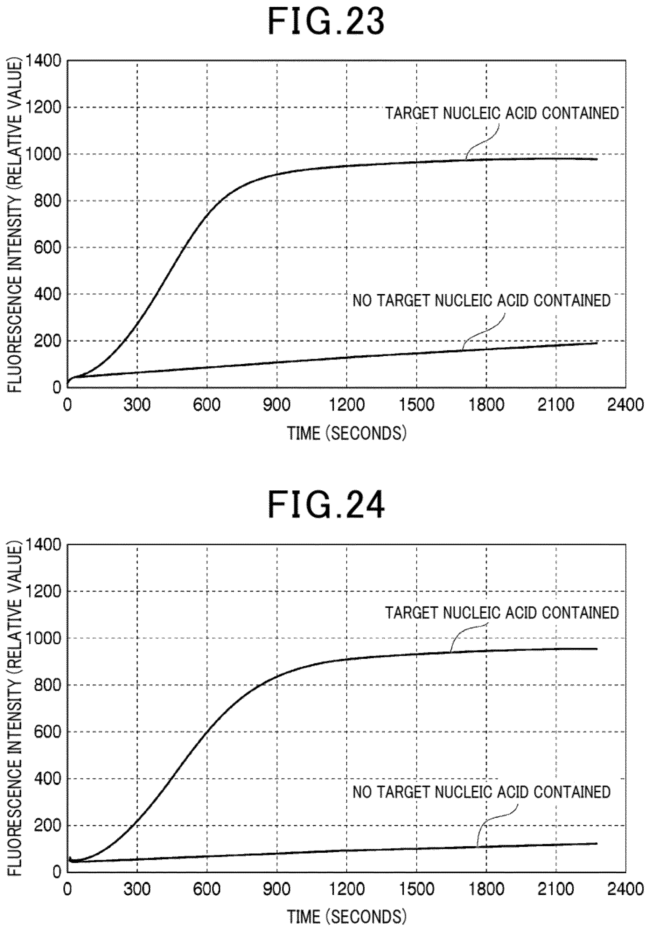

Fig. 23 is a graph showing the results of measuring the change in fluorescence intensity when fluorescent substrate B7 was used. Further, Fig. 24 is a graph showing the results of measuring the change in fluorescence intensity when fluorescent substrate B8 was used. In Figs. 23 and 24, the horizontal axis represents the time (seconds) elapsed from the start of reaction, and the vertical axis represents the fluorescence intensity (relative value).

The results show that the use of fluorescent substrate B8, in which both the fluorescent substance and the quenching substance are present in one strand of the double-stranded oligonucleotide portion of the hairpin structure, has higher reactivity in ICA.

### [Experimental Example B5]

### (Fluorescent Substrate Study 5)

ICA reactions were performed using a plurality of fluorescent substrates modified with fluorescent substances having different molecular weights to determine the S/N ratio, and the relationship between the molecular weight of the fluorescent substance and the S/N ratio was studied.

The target nucleic acid (SEQ ID NO: 23), the flap probe (SEQ ID NO: 24) and the invasion probe (SEQ ID NO: 25) were the same as those used in Experimental Example B1, and ICA was performed using the fluorescent substrates shown in Table 16 below.

**[Table 16]**

| Name | SEQ ID NO | Base sequence (5' → 3') |
|---|---|---|
| Fluorescent substrates B9 to B16 | 35 | F-**T**CT-T(Q)-AGCCGGTTTTCCGGCTGAGACCTCGGCGCG |

Fluorescent substrates B9 to B16 (SEQ ID NO: 35) shown in Table 16 were fluorescent substrates in which a quenching substance was located on the 3' side of a fluorescent substance, and specifically had a structure shown in the following formula (B13). In the following formula (B13), the fluorescent substance F modifies the base of the thymine residue at the 5' end, and Q modifies the base of the thymine residue in the chain.

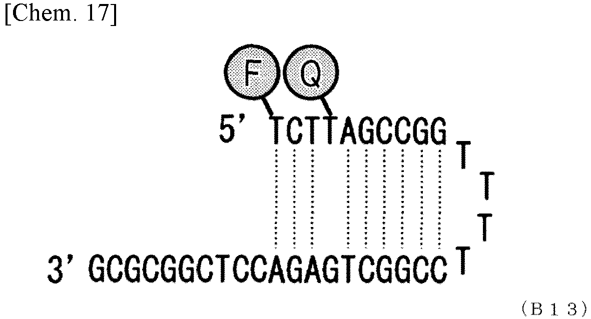

The combinations of the fluorescent substance F and the quenching substance Q in the fluorescent substrates B9 to B16 are shown in Table 17 below. Table 17 below also shows the molecular weights of the fluorescent substances and the S/N ratios measured by the method described below.

**[Table 17]**

| Name | Fluorescent substance | Molecular weight of fluorescent substance | Quenching substance | S/N ratio |
|---|---|---|---|---|
| Fluorescent substrate B9 | ATTO 425 | 401.45 | BHQ (registered trademark)-0 | 1.5 |
| Fluorescent substrate B10 | Alexa 488 | 534.47 | BHQ (registered trademark)-1 | 3.5 |
| Fluorescent substrate B11 | ATTO 542 | 914 | BHQ (registered trademark)-1 | 3.6 |
| Fluorescent substrate B12 | Yakima Y | 718.33 | BHQ (registered trademark)-1 | 3.7 |
| Fluorescent substrate B13 | Redmond R | 445.3 | BHQ (registered trademark)-2 | 3.3 |
| Fluorescent substrate B14 | ATTO 643 | 836 | BHQ (registered trademark)-2 | 5.2 |
| Fluorescent substrate B15 | Alexa 647 | 860 | BHQ (registered trademark)-2 | 5.5 |
| Fluorescent substrate B16 | Alexa 680 | 1,050 | BHQ (registered trademark)-2 | 2.5 |

When the nucleic acid fragment (SEQ ID NO: 26) excised from the flap probe (SEQ ID NO: 24) hybridizes to fluorescent substrates B9 to B16, the T (thymine) residues shown in bold in fluorescent substrates B9 to B16 forms a triplex structure that serves as a substrate for flap endonuclease, which is then cleaved. As a result, the fluorescent substance is separated from the quenching substance, and a fluorescence signal is detected upon irradiation with excitation light.

First, reaction solutions were prepared with the compositions shown in Table 18, and introduced into the wells of a fluidic device having a well array. The well array had approximately 930,000 wells, and the volume per well was approximately 1,683 fL.

Then, the fluidic device was set in an aluminum block bath (model "DTU-Mini," TAITEC Corporation), heated at 66°C for 25 minutes, and then observed using a microscope (product name "All-in-One fluorescence microscope," model "BZ-X810," KEYENCE Corporation).

Then, based on the microscopic observation images, luminescent wells (S) and non-luminescent wells (N) were identified, and luminance of the luminescent wells and non-luminescent wells was calculated. Then, the ratio of the luminance of the luminescent wells and the luminance of the non-luminescent wells, that is, the S/N ratio was calculated.

**[Table 18]**

| Reagent | Final concentration |
|---|---|
| Target nucleic acid | 1 µM |
| Flap probe | 1 µM |
| Invasion probe | 1 µM |
| Any of fluorescent substrates B9 to B16 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

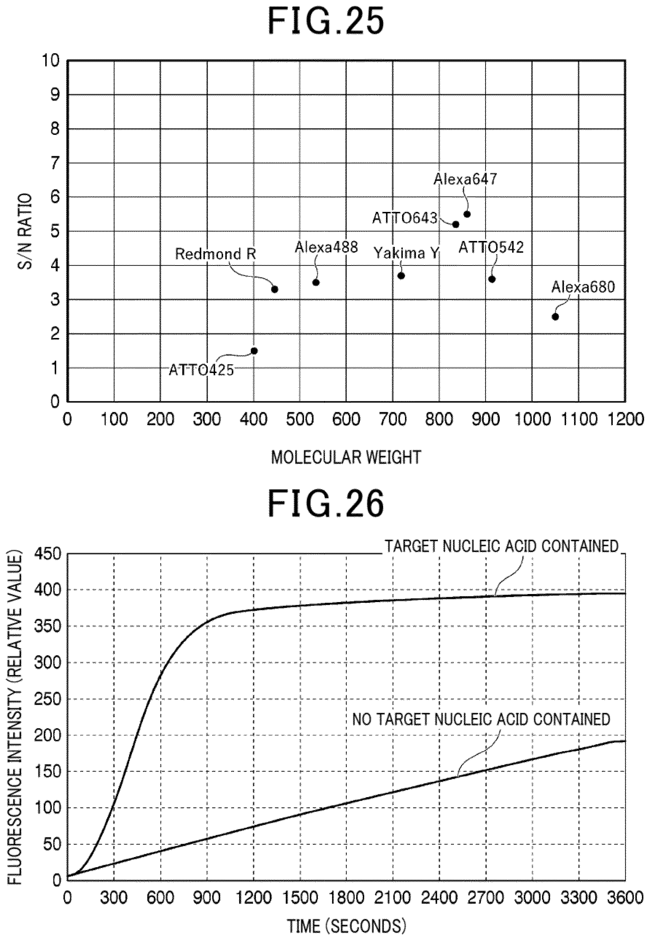

The calculated S/N ratios are shown in Table 17 above. Further, Fig. 25 is a graph summarizing the relationship between the molecular weights of the fluorescent substances and the S/N ratios. As a result, the S/N ratio is found to be 1.5 or higher when the molecular weight of the fluorescent substance is 350 to 1,100. Further, the S/N ratio is found to be 2 or higher when the molecular weight of the fluorescent substance is 445 to 1,100. Further, the S/N ratio is found to be 3 or higher when the molecular weight of the fluorescent substance is 445 to 914.

### [Experimental Example C1]

### (Fluorescent Substrate Study)

ICA was performed using a fluorescent substrate that does not contain a non-complementary base pair within the hairpin structure, and a fluorescent substrate that contains a non-complementary base pair within the hairpin structure, and reactivity was compared.

ICA was performed using the nucleic acid fragments shown in Tables 19 and 20 below. The target nucleic acid (SEQ ID NO: 1) in Table 19 was the nucleic acid to be detected. In this example, ICA detected the presence of g (guanine) indicated by a lowercase letter in the target nucleic acid. The flap probe (SEQ ID NO: 2) and the invasion probe (SEQ ID NO: 3) each had a base sequence complementary to the target nucleic acid (SEQ ID NO: 1).

When the flap probe (SEQ ID NO: 37) and the invasion probe (SEQ ID NO: 38) each hybridize to the target nucleic acid (SEQ ID NO: 36), a triplex structure is formed. Here, the lowercase portion "5'-cgcgccgaggc-3"' (SEQ ID NO: 39) in the flap probe (SEQ ID NO: 37) does not form a base pair, and forms a flap site.

The flap endonuclease recognizes the above triplex structure and cleaves the flap probe (SEQ ID NO: 37), whereby the nucleic acid fragment (SEQ ID NO: 39) is excised.

**[Table 19]**

| Name | SEQ ID NO | Base sequence (5' → 3') |
|---|---|---|
| Target nucleic acid | 36 | CCGAAGGGCATGAGCTGCgTGATGAGCTGCACGGTGGAGGTG AGGCAGATGCCCAGCAGGCGGCACACGTGGGGGTTGTCCACG CTGGCCATCACGTAGGCTTCC |
| Flap probe | 37 | cgcgccgaggcGCAGCTCATGCCC |
| Invasion probe | 38 | CCACCGTGCAGCTCATCAA |
| Nucleic acid fragment | 39 | cgcgccgaggc |

**[Table 20]**

| Name | SEQ ID NO | Base sequence (5' - 3') |
|---|---|---|
| Fluorescent substrate C1 | 40 | F-**T**CT-T(Q)-AGCCGGTTTTCCGGCT**A**AGACCTCGGCGCG |
| Fluorescent substrate C2 | 41 | F-**T**CT-T(Q)-AGCCGGTTTTCCGGCT**G**AGACCTCGGCGCG |

Fluorescent substrate C1 (SEQ ID NO: 40) shown in Table 20 was a fluorescent substrate that does not contain a non-complementary base pair within the hairpin structure, and specifically had a structure shown in the following formula (C1). In fluorescent substrate C1, F indicates fluorescein, and Q indicates BHQ (registered trademark)-1. As shown in the following formula (C1), fluorescent substrate C1 does not contain a non-complementary base pair within the hairpin structure.

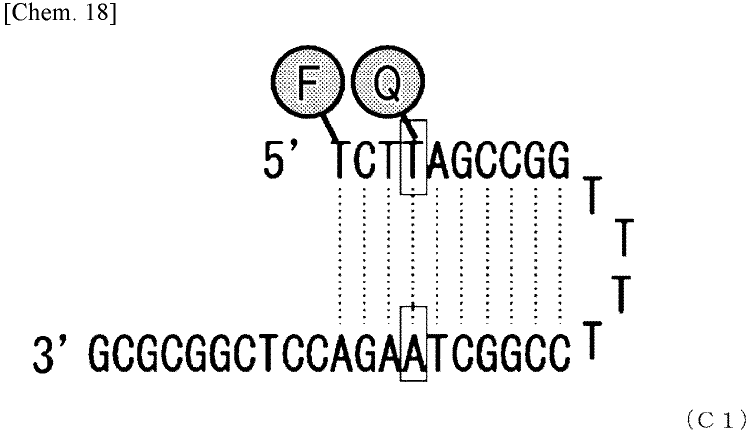

Fluorescent substrate C2 (SEQ ID NO: 41) shown in Table 20 was a fluorescent substrate that contains a non-complementary base pair within the hairpin structure, and specifically had a structure shown in the following formula (C2). In fluorescent substrate C2, F indicates fluorescein, and Q indicates BHQ (registered trademark)-1. As shown in the following formula (C2), fluorescent substrate C2 contains a non-complementary base pair within the hairpin structure. In the following formula (C2), the T (thymine) residue and the G (guanine) residue each enclosed in a square are not complementary to each other.

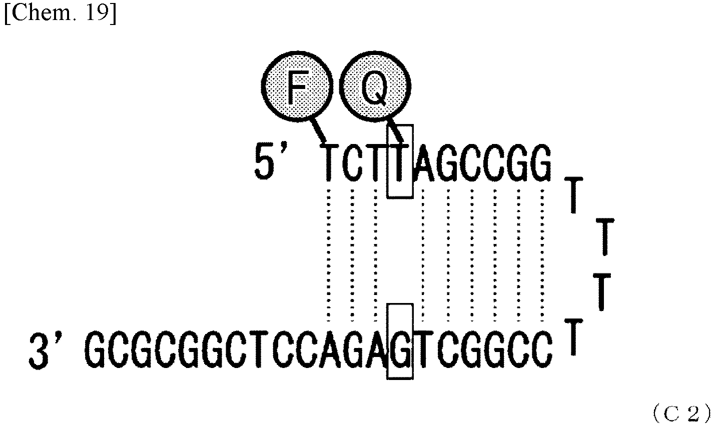

When the excised nucleic acid fragment (SEQ ID NO: 39) hybridizes to fluorescent substrate C1 or fluorescent substrate C2, the T (thymine) residue shown in bold in fluorescent substrate C1 or fluorescent substrate C2 in Table 20 forms a triplex structure that serves as a substrate for flap endonuclease, which is then cleaved. As a result, the fluorescent substance is separated from the quenching substance, and a fluorescence signal is detected upon irradiation with excitation light.

First, a reaction solution was prepared with the composition shown in Table 21 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 21]**

| Reagent | Final concentration |
|---|---|
| Target nucleic acid | 1 µM |
| Flap probe | 1 µM |
| Invasion probe | 1 µM |
| Fluorescent substrate C1 or fluorescent substrate C2 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

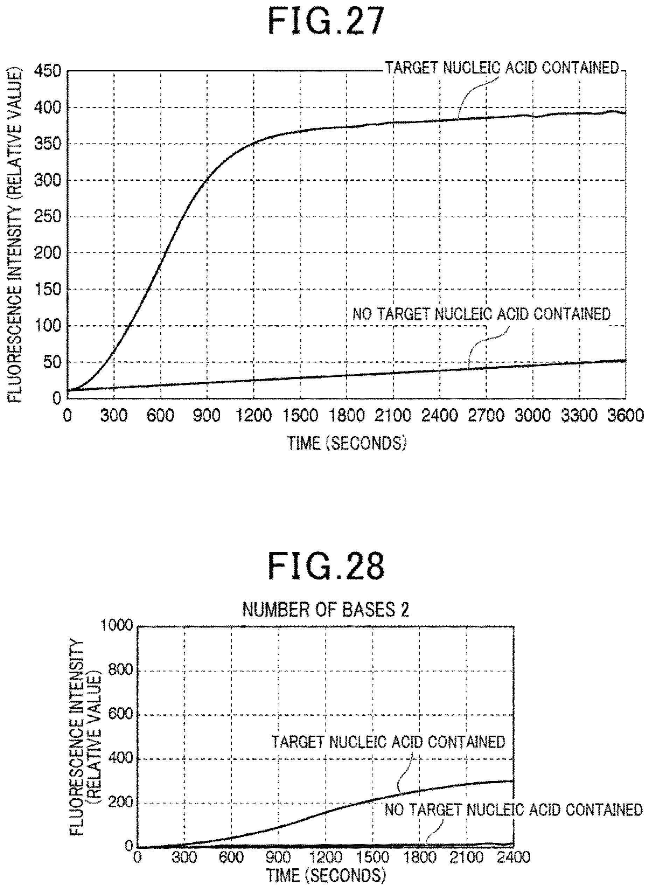

Fig. 26 is a graph showing the results of measuring the change in fluorescence intensity when fluorescent substrate C1 was used. Further, Fig. 27 is a graph showing the results of measuring the change in fluorescence intensity when fluorescent substrate C2 was used. In Figs. 26 and 27, the horizontal axis represents the time (seconds) elapsed from the start of reaction, and the vertical axis represents the fluorescence intensity (relative value).

The results show that the use of fluorescent substrate C2, which contains a non-complementary base pair within the hairpin structure, significantly suppresses an increase in background in ICA compared to the use of fluorescent substrate C1.

### [Experimental Example D1]

### (Fluorescent Substrate Study)

ICA was performed using fluorescent substrates in which the distance between the nucleotide residue labeled with a fluorescent substance and the nucleotide residue labeled with a quenching substance was varied, and reactivity was compared.

ICA was performed using the nucleic acid fragments shown in Tables 22 and 23 below. The target nucleic acid (SEQ ID NO: 42) in Table 22 was the nucleic acid to be detected. In this example, ICA detected the presence of g (guanine) indicated by a lowercase letter in the target nucleic acid. The flap probe (SEQ ID NO: 43) and the invasion probe (SEQ ID NO: 44) each had a base sequence complementary to the target nucleic acid (SEQ ID NO: 42).

When the flap probe (SEQ ID NO: 43) and the invasion probe (SEQ ID NO: 44) each hybridize to the target nucleic acid (SEQ ID NO: 42), a triplex structure is formed. Here, the lowercase portion "5'-cgcgccgaggc-3 "' (SEQ ID NO: 45) in the flap probe (SEQ ID NO: 43) does not form a base pair, and forms a flap site.

The flap endonuclease recognizes the above triplex structure and cleaves the flap probe (SEQ ID NO: 43), whereby the nucleic acid fragment (SEQ ID NO: 45) is excised.

**[Table 22]**

| Name | SEQ ID NO | Base sequence (5' → 3') |
|---|---|---|
| Target nucleic acid | 42 | CCGAAGGGCATGAGCTGCgTGATGAGCTGCACGGTGGAGGTG AGGCAGATGCCCAGCAGGCGGCACACGTGGGGGTTGTCCAC GCTGGCCATCACGTAGGCTTCC |
| Flap probe | 43 | cgcgccgaggcGCAGCTCATGCCC |
| Invasion probe | 44 | CCACCGTGCAGCTCATCAA |
| Nucleic acid fragment | 45 | cgcgccgaggc |

**[Table 23]**

| Name | SEQ ID NO | Base sequence (5' → 3') |
|---|---|---|
| Fluorescent substrate D1 | 46 | F-CT-T(Q)-AGCCGGTTTTCCGGCTGAGACCTCGGCGCG |
| Fluorescent substrate D2 | 47 | F-**T**CT-T(Q)-AGCCGGTTTTCCGGCTGAGACCTCGGCGCG |
| Fluorescent substrate D3 | 48 | F-T**T**CT-T(Q)-AGCCGGTTTTCCGGCTGAGACCTCGGCGCG |
| Fluorescent substrate D4 | 49 | F-TT**T**CT-T(Q)-AGCCGGTTTTCCGGCTGAGACCTCGGCGCG |

Fluorescent substrate D1 (SEQ ID NO: 46) shown in Table 23 was a fluorescent substrate in which the distance between the nucleotide residue labeled with a fluorescent substance and the nucleotide residue labeled with a quenching substance was 2 residues, and specifically had a structure shown in the following formula (D1). In fluorescent substrate D1, F indicates fluorescein, and Q indicates BHQ (registered trademark)-1. Further, as shown in the following formula (D1), fluorescent substrate 1 contains a non-complementary base pair within the hairpin structure.

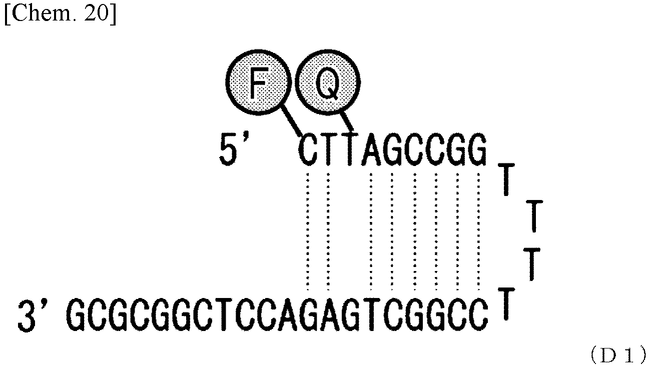

Fluorescent substrate D2 (SEQ ID NO: 47) shown in Table 23 was a fluorescent substrate in which the distance between the nucleotide residue labeled with a fluorescent substance and the nucleotide residue labeled with a quenching substance was 3 residues, and specifically had a structure shown in the following formula (D2). In fluorescent substrate D2, F indicates fluorescein, and Q indicates BHQ (registered trademark)-1. Further, as shown in the following formula (D2), fluorescent substrate D2 contains a non-complementary base pair within the hairpin structure.

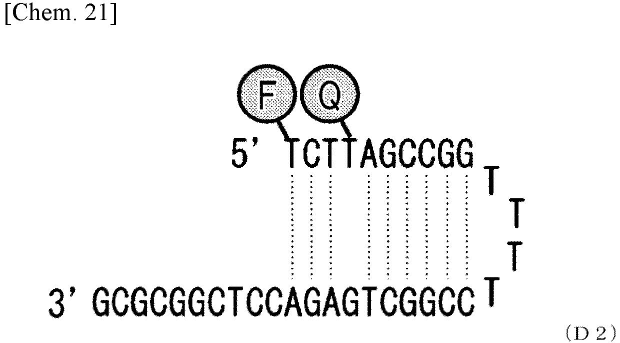

Fluorescent substrate D3 (SEQ ID NO: 48) shown in Table 23 was a fluorescent substrate in which the distance between the nucleotide residue labeled with a fluorescent substance and the nucleotide residue labeled with a quenching substance was 4 residues, and specifically had a structure shown in the following formula (D3). In fluorescent substrate D3, F indicates fluorescein, and Q indicates BHQ (registered trademark)-1. Further, as shown in the following formula (D3), fluorescent substrate D3 contains a non-complementary base pair within the hairpin structure.

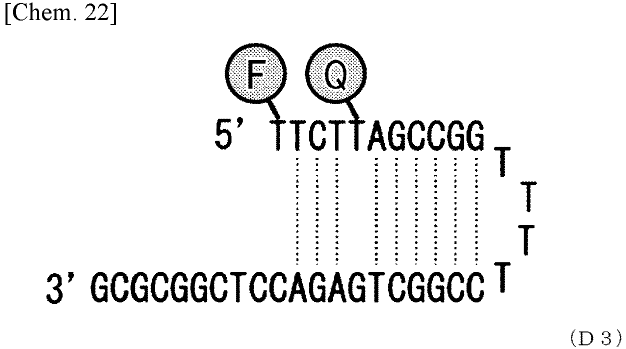

Fluorescent substrate D4 (SEQ ID NO: 49) shown in Table 23 was a fluorescent substrate in which the distance between the nucleotide residue labeled with a fluorescent substance and the nucleotide residue labeled with a quenching substance was 5 residues, and specifically had a structure shown in the following formula (D4). In fluorescent substrate D4, F indicates fluorescein, and Q indicates BHQ (registered trademark)-1. Further, as shown in the following formula (D4), fluorescent substrate D4 contains a non-complementary base pair within the hairpin structure.

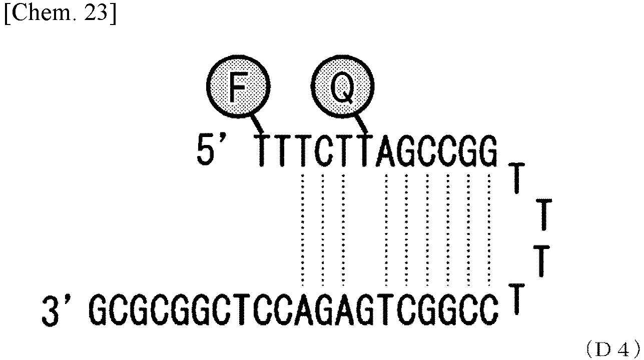

When the excised nucleic acid fragment (SEQ ID NO: 45) hybridizes to fluorescent substrate D1, the C (cytosine) residue shown in bold in fluorescent substrate 1 in Table 23 is cleaved due to fluctuations in substrate recognition ability of the flap endonuclease. As a result, the fluorescent substance is separated from the quenching substance, and a fluorescence signal is detected upon irradiation with excitation light. In fluorescent substrate D1, when cleaved by the flap endonuclease, the released oligonucleotide fragment has the length of 1 residue.

When the excised nucleic acid fragment (SEQ ID NO: 45) hybridizes to fluorescent substrates D2, D3 and D4, the T (thymine) residue shown in bold in fluorescent substrates D2, D3 and D4 in table 23 forms a triplex structure that serves as a substrate for flap endonuclease, which is then cleaved. As a result, the fluorescent substance is separated from the quenching substance, and a fluorescence signal is detected upon irradiation with excitation light. In fluorescent substrates D2, D3 and D4, when cleaved by the flap endonuclease, the released oligonucleotide fragment has the length of 1, 2 and 3 residues, respectively.

First, a reaction solution was prepared with the composition shown in Table 24 below, and introduced into a micro test tube. Then, the tube was set in a real-time PCR device, and after heating at 66°C for 60 minutes, the change in fluorescence intensity (excitation wavelength 490 nm, fluorescence wavelength 520 nm) was measured over time. Further, for comparison, a reaction solution was prepared by adding sterile water instead of the target nucleic acid, and the same measurements were performed.

**[Table 24]**

| Reagent | Final concentration |
|---|---|
| Target nucleic acid | 1 µM |
| Flap probe | 1 µM |
| Invasion probe | 1 µM |
| Any of fluorescent substrates D1 to D4 | 2 µM |
| 3-morpholinopropanesulfonic acid (pH 7.9) | 10 mM |
| MgCl₂ | 10 mM |
| Tween 20 | 0.05 (v/v)% |
| Flap endonuclease | 1,000 U |
| Sterile water | appropriate amount |
| Total | 10 µL |

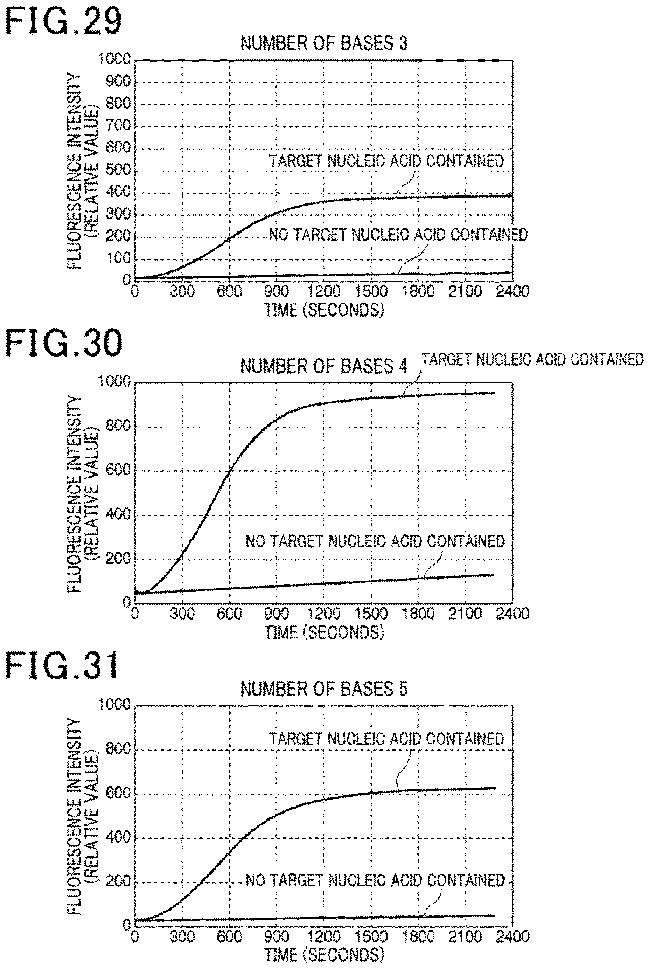

Fig. 28 is a graph showing the results of measuring the change in fluorescence intensity when fluorescent substrate D1 was used. Fig. 29 is a graph showing the results of measuring the change in fluorescence intensity when fluorescent substrate D2 was used. Fig. 30 is a graph showing the results of measuring the change in fluorescence intensity when fluorescent substrate D3 was used. Fig. 31 is a graph showing the results of measuring the change in fluorescence intensity when fluorescent substrate D4 was used. In Figs. 28 to 31, the distance between the nucleotide residue labeled with a fluorescent substance and the nucleotide residue labeled with a quenching substance in each fluorescent substrate is 2, 3, 4 and 5 residues, respectively.

The results show that, the efficiency of cleavage of the fluorescent substrate by the flap endonuclease is high when the distance between the nucleotide residue labeled with a fluorescent substance and the nucleotide residue labeled with a quenching substance in a fluorescent substrate is 4 or more residues, and is particularly high when the distance is 4 residues.

### [Industrial Applicability]

According to the present invention, it is possible to provide a technique for improving the reaction efficiency in reactions for detecting trace amounts of analyte molecules.

### [Reference Signs List]

- 100: Target nucleic acid,
- 101: Base,
- 110: Flap probe,
- 120: Invasion probe,
- 130: First triplex structure,
- 140, 170: Flap site (nucleic acid fragment),
- 141: Region,
- 150: Nucleic acid fragment,
- 151: Mismatch site,
- 160: Second triplex structure,
- F: Fluorescent substance,
- Q: Quenching substance.

## Claims

1. A single-stranded oligonucleotide labeled with a fluorescent substance and a quenching substance, wherein
a hairpin structure is formed by self-hybridization on the 5' side, and when a single-stranded oligonucleotide having a complementary base sequence on the 3' side hybridizes, a triplex structure is formed on the 5' end portion, and cleaved by a flap endonuclease that recognizes the triplex structure, whereby the fluorescent substance is released from the quenching substance, the fluorescent substance emitting fluorescence when irradiated with excitation light, and
the single-stranded nucleotide satisfies one of the following conditions (A) to (C):
(A) a single-stranded oligonucleotide including a non-complementary base pair within the hairpin structure;
(B) a single-stranded oligonucleotide in which a nucleotide residue labeled with the fluorescent substance and a nucleotide residue labeled with the quenching substance are separated from each other by 4 or more residues; and
(C) a single-stranded oligonucleotide in which the fluorescent substance is bound to a portion other than the base.

2. The single-stranded oligonucleotide according to claim 1, which satisfies the condition (A), wherein
the fluorescent substance is labeled at a 5' end nucleotide residue, and
the quenching substance is labeled at a nucleotide residue located closer to the 5' side among nucleotide residues constituting the non-complementary base pair.

3. The single-stranded oligonucleotide according to claim 1, wherein
the base of the 5' end nucleotide residue is a pyrimidine base.

4. The single-stranded oligonucleotide according to claim 1, wherein
the quenching substance is labeled on the 3' side of the fluorescent substance, and
an oligonucleotide fragment containing the fluorescent substance is released when cleaved by a flap endonuclease.

5. The single-stranded oligonucleotide according to claim 1, wherein
the fluorescent substance is labeled at a 5' end nucleotide residue,
an oligonucleotide fragment containing the fluorescent substance is released when cleaved by a flap endonuclease, and
the oligonucleotide fragment has a length of 2 or more residues.

6. The single-stranded oligonucleotide according to claim 1, wherein
a phosphate group is added to the 5' end, and the fluorescent substance is bound to the phosphate group.

7. The single-stranded oligonucleotide according to claim 1, wherein
the quenching substance is bound to a base of a nucleotide residue located on the 3' side of the fluorescent substance.

8. The single-stranded oligonucleotide according to claim 1, wherein
the fluorescent substance has a molecular weight of 350 to 1,100.

9. The single-stranded oligonucleotide according to claim 1, wherein
both the fluorescent substance and the quenching substance are present in one strand of a double-stranded oligonucleotide portion of the hairpin structure.

10. A method for detecting fluorescence from a substrate including a fluorescent substance, a quenching substance and a substrate body, the method comprising:
a step of causing the fluorescent substance to emit fluorescence by separating the fluorescent substance from the quenching substance in the substrate, wherein
when the fluorescent substance is in spatial proximity to the quenching substance, fluorescence emission from the fluorescent substance is suppressed by the quenching substance, and
a peak wavelength of an absorption spectrum of the quenching substance is on a shorter wavelength side of a peak wavelength of an emission spectrum of the fluorescent substance.

11. The method according to claim 10, wherein
the step of causing the fluorescent substance to emit fluorescence is performed in the presence of a target substance.

12. The method according to claim 10 or 11, wherein
the peak wavelength of the absorption spectrum of the quenching substance is 50 nm to 100 nm shorter than the peak wavelength of the emission spectrum of the fluorescent substance.

13. The method according to claim 10 or 11, wherein
the substrate body is a nucleic acid.

14. The method according to claim 10 or 11, wherein
the peak wavelength of the emission spectrum of the fluorescent substance is 600 nm to 700 nm, and
the peak wavelength of the absorption spectrum of the quenching substance is 550 nm to 640 nm.

15. The method according to claim 14, wherein
in the step of causing the fluorescent substance to emit fluorescence, the fluorescent substance is separated from the quenching substance in the substrate by the action of a nucleic acid cleaving enzyme.

16. The method according to claim 10 or 11, wherein
the step of causing the fluorescent substance to emit fluorescence is performed in a microspace.

17. The method according to claim 11, wherein
the target substance is a nucleic acid.

18. The method according to claim 10 or 11, further comprising
a step of observing the fluorescence with a fluorescence microscope.
